# EUROPEAN PATENT SPECIFICATION

(11) **EP 2 548 959 B1**
(45) Date of publication and mention of the grant of the patent: **18.07.2018**
(21) Application number: 11756406.2
(22) Date of filing: 17.03.2011
(51) Int. Cl.: C12N 15/09, A01H 1/00, A01H 5/02, C07K 14/415, C30B 7/14, C30B 29/58, G01N 33/53, G06F 19/16, C12N 15/82

(54) **FLORIGEN-ACTIVATING COMPLEX**
FLORIGEN-AKTIVIERENDER KOMPLEX
COMPLEXE ACTIVATEUR DU FLORIGÈNE

(30) Priority: 17.03.2010 JP 2010061562
(43) Date of publication of application: 23.01.2013
(73) Proprietor: National University Corporation Nara Institute of Science and Technology, Ikoma-shi, Nara 630-0192 (JP)
(72) Inventor: OHKI, Izuru, Ikoma-shi Nara 630-0192 (JP); TAOKA, Ken-ichiro, Ikoma-shi Nara 630-0192 (JP); TSUJI, Hiroyuki, Ikoma-shi Nara 630-0192 (JP); KOJIMA, Chojiro, Suita-shi Osaka 565-0871 (JP); SHIMAMOTO, Ko, Ikoma-shi Nara 630-0192 (JP)
(74) Representative: Krauss, Jan
(86) International application number: PCT/JP2011/056426
(87) International publication number: WO 2011/115222

(56) References cited:
- WO-A1-02/42475
- WO-A1-99/53070
- WO-A2-2004/022755
- WO-A2-2004/061122
- DATABASE UniProt [Online] 27 September 2005 (2005-09-27), "SubName: Full=Flowering locus T; SubName: Full=VRN3;", XP002716926, retrieved from EBI accession no. UNIPROT:Q3ZPM9 Database accession no. Q3ZPM9 -& YAN L ET AL: "The wheat and barley vernalization gene VRN3 is an orthologue of FT", PROCEEDINGS OF THE NATIONAL ACADEMY OF SCIENCES OF THE UNITED STATES OF AMERICA, vol. 103, no. 51, December 2006 (2006-12), pages 19581-19586, XP002716927, ISSN: 0027-8424
- PURWESTRI YEKTI ASIH ET AL: "The 14-3-3 Protein GF14c Acts as a Negative Regulator of Flowering in Rice by Interacting with the Florigen Hd3a", PLANT AND CELL PHYSIOLOGY, vol. 50, no. 3, March 2009 (2009-03), pages 429-438, XP002716928, ISSN: 0032-0781
- TAMAKI SHOJIRO ET AL: "Hd3a protein is a mobile flowering signal in rice", SCIENCE (WASHINGTON D C), vol. 316, no. 5827, May 2007 (2007-05), pages 1033-1036, XP002716929, ISSN: 0036-8075
- KEN'ICHIRO TAOKA ET AL.: 'Target Tanpaku Kenkyu Program no Seika, Structural aspects of florigen to understand the molecular mechanism of flowering' PROTEIN, NUCLEIC ACID AND ENZYME vol. 54, no. 12, 10 September 2009, pages 1702 - 1707, XP008167160
- HANZAWA Y. ET AL.: 'A single amino acid converts a repressor to an activator of flowering.' PROC. NATL.ACAD.SCI. USA. vol. 102, no. 21, May 2005, pages 7748 - 7753, XP002633034
- HIROYUKI TSUJI ET AL.: 'Ine Florigen Hd3a Tanpakushitsu to Sogo Sayo suru Tensha Inshi OsFDl no Kino Kaiseki' PROCEEDINGS OF THE ANNUAL MEETING OF THE JAPANESE SOCIETY OF PLANT PHYSIOLOGISTS vol. 51, 12 March 2010, page 214, XP008167168
- CHEN F. ET AL.: 'The rice 14-3-3 gene family and its involvement in responses to biotic and abiotic stress.' DNA RES. vol. 13, no. 2, April 2006, pages 53 - 63, XP055099979

## Description

The present invention relates to a method of regulating the flowering of a plant by suppressing the formation of a florigen activation complex comprising a complex of a florigen, a 14-3-3 protein, and a bZIP transcription factor.

### DESCRIPTION

Regulation of flowering of plants under variuous environments is considered to be effective for realizing stable food production and establishment of a recycling-oriented economic society system utilizing plant biomass and the like. A molecular basis for signal transduction of a plant environment response needs to be elucidated in order to establish a technology for regulating flowering of plants.

It was proposed 70 years ago that a florigen was present as an inducer of flowering of plants. In recent years, it has been clarified that the molecular nature for the florigen is a product of an FT gene universally present in higher plants. The florigen is a molecule which is synthesized in leaves when day length becomes optimum for flower-bud formation, moves to the shoot apex, and initiates flowering. In rice, Hd3a corresponds to the florigen and is considered to promote heading, and there has been proposed use of a rice Hd3a gene for regulating flowering of plants (Patent Literature 1 and Non Patent Literature 1).

There is a report that rice Hd3a interacts with a rice 14-3-3 protein GF14c, thereby suppressively controlling flowering (Non Patent Literature 2). In Non Patent Literature 2, it has been found that the rice 14-3-3 protein GF14c interacts with Hd3a, as a result of screening of a protein which interacts with Hd3a by a yeast two-hybrid method. It has been suggested that GF14c is mainly localized in the cytoplasm and a complex of Hd3a and GF14c is mainly present in the cytoplasm. GF14c has been considered to act as a negative regulator of flowering based on, for example, the fact that overexpression of GF14c leads to a delay in flowering.

In *Arabidopsis*, it is considered that an FT protein as the molecular nature for the florigen interacts with a bZIP transcription factor FD and the like in the nucleus, contributing to expression of floral meristem identity genes (Non Patent Literatures 3 and 4). There is a report on use of a bZIP transcription factor for controlling flowering (Patent Literature 2).

Hanzawa *et al.* (Non Patent Literature 5) disclose that a single amino acid exchange can convert a repressor protein to an activator protein of flowering.

Yan *et al.* (Non Patent Literature 6) disclose the sequences of wild type florigen polypeptides.

WO 2004/022755 A2 (Patent Literature 3) further discloses the sequences of wild type florigen polypeptides.

WO 99/53070 A1 (Patent Literature 4) discloses methods for producing genetically engineered plants. Although there are many research reports on the florigen, an action mechanism of the florigen in floral induction has not been clarified yet.

### Citation List

### Patent Literature

[PTL 1] JP 2002-153283 A
[PTL 2] JP 2003-274972 A
[PTL 3] WO 2004/022755 A2
[PTL4] WO 99/53070

### Non Patent Literature

[NPL 1] Tamaki S et al. Science (2007) vol. 316 (5827) pp. 1033-6
[NPL 2] Purwestri YA et al. Plant and Cell Physiology 2009 50(3): 429-438
[NPL 3] Wigge et al. Science (2005) vol. 309 (5737) pp. 1056-9
[NPL 4] Abe et al. Science (2005) vol. 309 (5737) pp. 1052-6
[NPL 5] Hanzawa et al. Proceedings of the National Academy of Sciences (2005) vol. 102 (21) pp. 7748-7753
[NPL 6] Yan et al. Proceedings of the National Academy of Sciences 2006 vol 103 (51) pp. 19581-19586

### Summary of Invention

### Technical Problem

The object of the present invention is to provide a crystal of a florigen activation complex, including a florigen bound to a bZIP transcription factor via a 14-3-3 protein. Another object of the present invention is to regulate flowering of a plant by clarifying mechanisms of interactions among those proteins utilizing the crystal and controlling such interactions.

### Solution to Problem

The present invention is characterized by the appended set of claims.

### Advantageous Effects of Invention

The crystal of the present disclosure provides conformational information on a florigen activation complex important for the elucidation of a mechanism for regulating flowering of plants. In addition, the crystal may be used as a material for additional research on the mechanism for regulating flowering. Further, it is possible to artificially regulate flowering of plants based on the conformational information obtained from the present disclosure. In the present invention, the binding site of each protein in the florigen activation complex has been clarified. Information on the binding site may be used as a novel target of floral regulation, which can contribute to efficienct floral regulation. A transgenic plant, which may be widely utilized for an increase of yield of an agricultural product, an improvement in efficiency of breeding, and the like, can be obtained based on the conformational information obtained in the present invention.

### Brief Description of Drawings

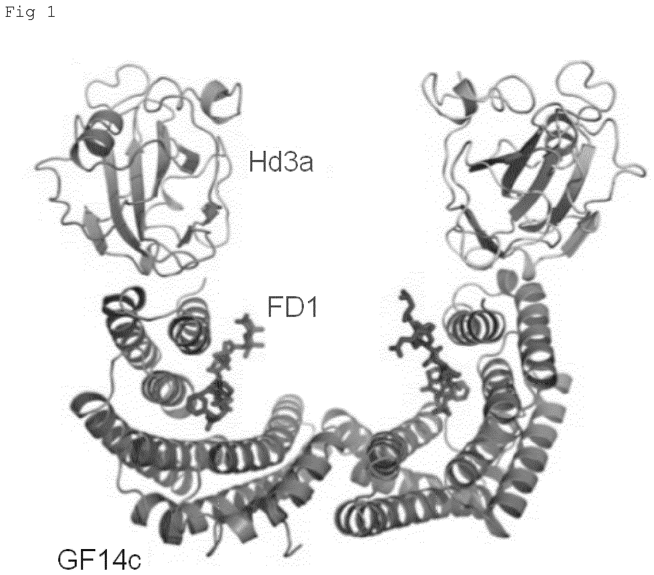

[FIG. 1] A view illustrating a conformation of a florigen activation complex including three proteins Hd3a, GF14c, and OsFD1.
[FIG. 2] An image showing the results of in vitro GST pull-down assays of three combinations, i.e., Hd3a and OsFD1, Hd3a and GF14c, GF14c and OsFD1 (Example 1(1)).
[FIG. 3] A graph showing the results of analysis of an interaction between Hd3a and GF14c using NMR (Example 1(2)).

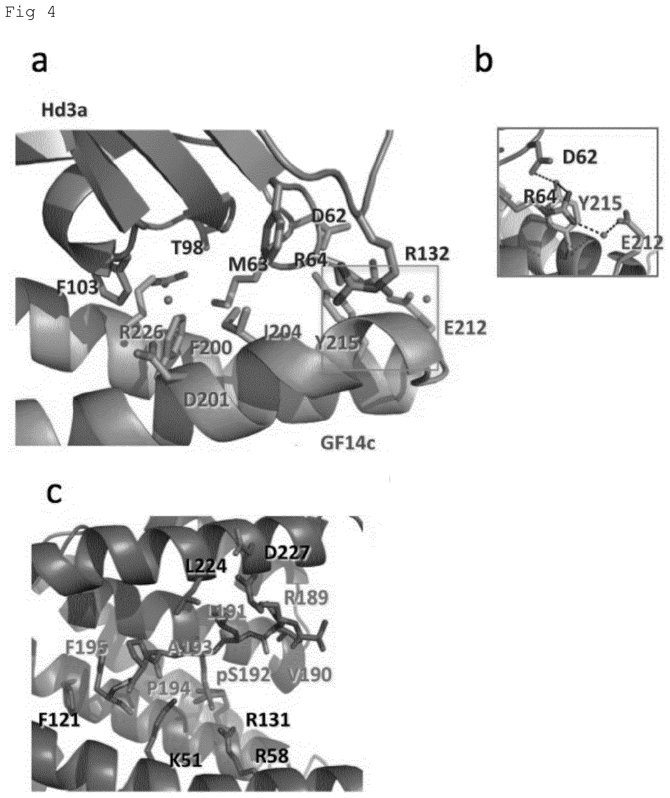

[FIGS. 4] Views illustrating the details of an interaction between Hd3a and GF14c. FIG. 4b is an expanded view illustrating the vicinity of Hd3a R64 (Example 3).
[FIGS. 5] Images showing the results of an in vitro GST pull-down assay with GF14c using mutant Hd3a (FIG. 5a) and the results of an in vitro GST pull-down assay with Hd3a using mutant GF14c (FIG. 5b) (Example 6).
[FIG. 6] An image showing the results of a gel-shift assay of a florigen activation complex including Hd3a, GF14c, and OsFD1 and C-box DNA (Example 7).
[FIG. 7] An image showing the results of confirmation of an interaction between each isoform of OsGF14 and Hd3a by a yeast two-hybrid method (Example 8).
[FIGS. 8] Images showing the results of confirmation of interactions among Hd3a, OsGF14b, and OsFD1 by a yeast two-hybrid method (Example 9).
[FIG. 9] An image showing the results of confirmation of interactions among Hd3a, OsGF14b, and OsFD1 in the shoot apex of rice by immunoprecipitation (Example 10).
[FIG. 10] An image showing the results of confirmation of subcellular localization of Hd3a, OsGF14b, and OsFD1 (Example 11).
[FIGS. 11] Images showing the results of confirmation of subcellular localization of Hd3a, OsGF14b, and and OsFD1 (Example 11).
[FIGS. 12] Images showing the results of confirmation of subcellular localization of Hd3a, OsGF14b, and OsFD1 (Example 11).
[FIGS. 13] Graphs showing the results of confirmation of an influence of transient expression of Hd3a, OsGF14b, and OsFD1 on OsMADS15 gene transcription (Example 12).
[FIG. 14] An image showing the results of confirmation of an influence of Hd3a mutation on flowering of plants (Example 13-1).
[FIG. 15] A view illustrating an action mechanism of a florigen activation complex, which is obtained by the present invention.
[FIG. 16] An image showing the results of confirmation of an influence of Hd3a mutation on flowering of plants (Example 13-2)

### Description of Embodiments

In general, a plant has a vegetative growth stage and a reproductive growth stage and forms flower buds upon the transition of a growth phase from vegetative growth to reproductive growth. This phenomenon of transition from vegetative growth to reproductive growth is referred to as "flowering." For example, the flowering in rice and wheats as monocotyledonous plants is heading. The heading refers to that the internode (panicle base) below the ear rapidly elongates and appears from the leaf sheath of the flag leaf. The time when the ear tip appears and the time when the ear including the base portion completely appears are defined as the heading in rice and wheats, respectively.

In the present invention, the plant means a higher plant having a florigen, and is preferably a short-day plant, which forms flower buds when sunshine duration per day is equal to or less than a predetermined time period, more preferably a monocotyledonous short-day plant, still more preferably a gramineous plant, most preferably rice.

### (Florigen activation complex)

The present disclosure relates to a florigen activation complex including a complex of a florigen, a 14-3-3 protein, and a bZIP transcription factor, in which the florigen is bound to the bZIP transcription factor via the 14-3-3 protein.

In the present invention, the florigen is not particularly limited, and examples thereof include rice Hd3a and RFT1 (Genbank Accession No. AB062676), *Arabidopsis* FT (Genbank Accession No. AB027504) and TSF (Genbank Accession No. AB027506), tomato SFT (Genbank Accession No. AY186735), and wheat VRN3 (Genbank Accession No. LOC100037541). Of those, rice Hd3a is preferred. Rice Hd3a includes a protein represented by an amino acid sequence set forth in SEQ ID NO: 1 (Os06g0157700) (Genbank Accession No. BAB61028: derived from *Oryza sativa* Japonica group cultivar Nipponbare), or a protein which is formed of an amino acid sequence having deletions, substitutions, additions, and/or insertions of one to several amino acid residues in the amino acid sequence set forth in SEQ ID NO: 1, and functions as a florigen. Rice Hd3a in the present invention is preferably a florigen polypeptide fragment which includes at least a sequence of amino acids at positions 62 to 132, starts with one of amino acids at positions 1 to 6, and ends with one of amino acids at positions 165 to 177 in SEQ ID NO: 1, more preferably a polypeptide fragment formed of a sequence of amino acids at positions 6 to 170 in SEQ ID NO: 1. Further, it is particularly preferred that the polypeptide fragment have mutations of C43L/C109S/C166S in SEQ ID NO: 1.

In the present invention, the 14-3-3 protein is not particularly limited but is preferably rice GF14. Rice GF14 has eight isoforms, i.e., OsGF14a (Os08g0480800), OsGF14b (Os04g0462500), OsGF14c (Os08g0430500), OsGF14d (Os11g0546900), OsGF14e (Os02g0580300), OsGF14f (Os03g0710800), OsGF14g (Os01g0209200), and OsGF14h (Os11g0609600). In the present invention, GF14b, GF14c, GF14e, or GF14f is preferred, and GF14c or GF14b is more preferred. It should be noted that, in this description, numbers beginning with Os are numbers of gene loci specified in the Rice annotation project database (RAP-DB), unless otherwise stated. Those numbers may be used for specifying amino acid sequences and base sequences of proteins such as GF14 isoforms.

Rice GF14c includes a protein represented by an amino acid sequence set forth in SEQ ID NO: 2 (Os08g0430500) (Genbank Accession No. AAB07457.1: derived from *Oryza sativa* Japonica group cultivar Nipponbare), or a protein which is formed of an amino acid sequence having deletions, substitutions, additions, and/or insertions of one to several amino acid residues in the amino acid sequence set forth in SEQ ID NO: 2, and functions as a 14-3-3 protein to bind to a florigen. The 14-3-3 protein in the present invention is preferably a polypeptide fragment which includes at least a sequence of amino acids at positions 51 to 227, starts with one of amino acids at positions 1 to 5, and ends with one of amino acids at positions 230 to 256 in SEQ ID NO: 2, more preferably a polypeptide fragment formed of a sequence of amino acids at positions 1 to 235 in SEQ ID NO: 2.

Further, rice GF14b includes a protein represented by an amino acid sequence set forth in SEQ ID NO: 4 (Os04g0462500) (Genbank Accession No. AK071822: derived from *Oryza sativa* Japonica group cultivar Nipponbare), or a protein which is formed of an amino acid sequence having deletions, substitutions, additions, and/or insertions of one to several amino acid residues in the amino acid sequence set forth in SEQ ID NO: 4, and functions as a 14-3-3 protein to bind to a florigen. Rice GF14b has an amino acid sequence shifted by 6 residues as compared to the amino acid sequence of GF14c. The sequence of amino acids at positions 51 to 227 in SEQ ID NO: 2 corresponds to a sequence of amino acids at positions 57 to 233 in SEQ ID NO: 4. The amino acid sequence which starts with one of amino acids at positions 1 to 5 and ends with one of amino acids at positions 230 to 256 in SEQ ID NO: 2 corresponds to an amino acid sequence which starts with one of amino acids at positions 7 to 11 and ends with one of amino acids at positions 236 to 262 in SEQ ID NO: 4.

In the present disclosure, the bZIP transcription factor is not particularly limited and examples thereof include rice OsFD1 and *Arabidopsis* FD (Genbank Accession No. AB105823). Of those, rice OsFD1 is preferred. Rice OsFD1 includes a protein represented by an amino acid sequence set forth in SEQ ID NO: 3 (Os09g0540800: derived from *Oryza sativa* Japonica group cultivar Nipponbare) (Rice genome annotation locus No. LOC_Os09g36910.1 specified in the TIGR Rice Genome Anootation Database), or a protein which is formed of an amino acid sequence having deletions, substitutions, additions, and/or insertions of one to several amino acid residues in the amino acid sequence set forth in SEQ ID NO: 3, and functions as a bZIP transcription factor. The bZIP transcription factor in the present disclosure is preferably a polypeptide fragment which includes at least a sequence of amino acids at positions 189 to 195, starts with one of amino acids at positions 182 to 188, and ends with the amino acid at position 195 in SEQ ID NO: 3, more preferably a polypeptide fragment formed of a sequence of amino acids at positions 187 to 195 in SEQ ID NO: 3. Further, in the polypeptide fragment, serine at position 192 is preferably phosphorylated. The phosphorylation of the serine is considered to be necessary for the binding of the 14-3-3 protein to the bZIP transcription factor.

The florigen, the 14-3-3 protein, and the bZIP transcription factor bind to each other to form a florigen activation complex. A region of a sequence of amino acids at positions 62 to 132 in SEQ ID NO: 1 or a region corresponding to the region in the florigen having an amino acid sequence other than SEQ ID NO: 1, and/or a region of a sequence of amino acids at positions 200 to 227 in SEQ ID NO: 2 or a region corresponding to the region in the 14-3-3 protein having an amino acid sequence other than SEQ ID NO: 2 are/is considered to be important for binding between the florigen and the 14-3-3 protein. Further, it has been clarified in the present disclosure that a region of a sequence of amino acids at positions 51 to 227 in SEQ ID NO: 2 or a region corresponding to the region in the 14-3-3 protein having an amino acid sequence other than SEQ ID NO: 2, and/or a region of a sequence of amino acids at positions 189 to 195 in SEQ ID NO: 3 or a region corresponding to the region in the bZIP transcription factor having an amino acid sequence other than SEQ ID NO: 3 are/is important for binding between the 14-3-3 protein and the bZIP transcription factor.

In addition, among those regions, amino acids of M63, R64, P96, F103, and R132 in SEQ ID NO: 1 or amino acids corresponding to the amino acids in the florigen having an amino acid sequence other than SEQ ID NO: 1, and/or amino acids of F200, D201, 1204, E212, Y215, R226, and D227 in SEQ ID NO: 2 or amino acids corresponding to the amino acids in the 14-3-3 protein having an amino acid sequence other than SEQ ID NO: 2 are considered to be important for binding between the florigen and the 14-3-3 protein. Further, it has been clarified in the present disclosure that amino acids of K51, R58, F121, R131, L224, and D227 in SEQ ID NO: 2 or amino acids corresponding to the amino acids in the 14-3-3 protein having an amino acid sequence other than SEQ ID NO: 2 are important for binding between the 14-3-3 protein and the bZIP transcription factor.

The "site of a protein corresponding to a particular amino acid (e.g., D62) in SEQ ID NO: X" to be used in the present invention means to include, in addition to a site of a particular amino acid (e.g., D62) in SEQ ID NO: X, a site corresponding to the particular amino acid (e.g., D62) in a protein having an amino acid sequence other than SEQ ID NO: X and having a function equivalent to that of a protein of SEQ ID NO: X. For example, the "sites of the 14-3-3 protein corresponding to F200, D201, 1204, E212, Y215, and R226 in SEQ ID NO: 2" include, in addition to sites of F200, D201, 1204, E212, Y215, and R226 in SEQ ID NO: 2, amino acids corresponding to the amino acids in the 14-3-3 protein having an amino acid sequence other than SEQ ID NO: 2, for example, F206, D207, 1210, E218, Y221, and R232 of rice GF14b (corresponding to F200, D201, 1204, E212, Y215, and R226 of rice GF14c).

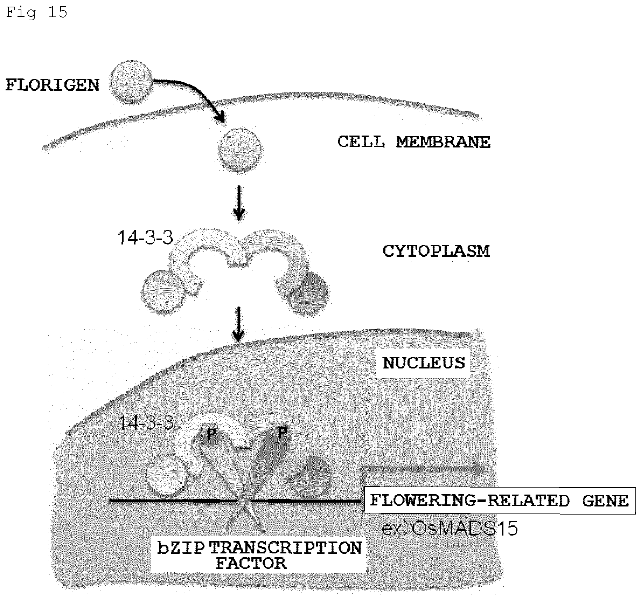

The action mechanism of a florigen activation complex in flowering is described (see FIG. 15). A 14-3-3 protein acts as an adaptor of a florigen transported from leaves to the shoot apex. When the florigen enters shoot apex cells, the florigen is considered to first bind to the 14-3-3 protein in the cytoplasm. At this stage, OsFD1 has been expressed in the shoot apex cells, and such complex of the florigen and the 14-3-3 protein enters the nucleus from the cytoplasm and binds to a bZIP transcription factor to form a florigen activation complex, which is retained in the nucleus. When the series of processes are initiated, the florigen activation complex is accumulated in the nucleus. After that, the florigen activation complex activates the transcription of a flowering-related gene (e.g., an OsMADS15 gene), which causes floral induction. In such action mechanism, the florigen is trapped by the 14-3-3 protein in the cytoplasm of the shoot apex cells, and the complex of the florigen and the 14-3-3 protein is efficiently transported by the bZIP transcription factor into the nucleus for the transcriptional activation of a gene necessary for floral induction. Further, the transcriptional activation of the flowering-related gene by the florigen activation complex in the present invention is considered to occur through the binding of the florigen activation complex to C-box DNA. C-box DNA is represented by a base sequence of GACGTC (SEQ ID NO: 10) and is present in a promoter region of the flowering-related gene. The florigen activation complex is estimated to form a stable complex on the C-box of the flowering-related gene and activate the transcription of the flowering-related gene. The florigen, the 14-3-3 protein, the bZIP transcription factor, mutant proteins thereof, a complex of the florigen and the 14-3-3 protein, a complex of the 14-3-3 protein and the bZIP transcription factor, and the florigen activation complex as a complex of the three proteins may be in such a state that they are isolated and purified from cells, tissues, and the like, or may be in such a state that a gene encoding a protein is introduced into host cells such as yeast and *Escherichia coli* and the protein is expressed in the cells. Further, the present disclosure also encompasses polynucleotides encoding those proteins. To those proteins, depending on expression systems of host cells, a peptide fragment may be added in such an amount that functions of the proteins are not affected. For example, in the case of using an expression system of *Escherichia coli,* an *Escherichia coli* expression plasmid-derived peptide fragment (GPGHM) has been added to the N-terminal of a protein.

The complex of the florigen and the 14-3-3 protein or the complex of the 14-3-3 protein and the bZIP transcription factor may be produced by bringing the two proteins into contact with each other in an isolated and purified state or in a living body. Similarly, the florigen activation complex as a complex of the three proteins may also be produced by bringing the three kinds of proteins or any one of the complexes of two proteins and the remaining protein into contact with each other in an isolated and purified state or in a living body such as cells.

### (Production method for crystal of florigen activation complex or florigen)

First, a protein solution is produced. A protein is allowed to be present in a solution formed of a buffer, a salt, a reducing agent, and the like. Any buffer, salt, and reducing agent may be used as long as the conformation of the protein is not affected. Examples of the buffer include 1 to 500 mM Na-HEPES, sodium phosphate, potassium phosphate, and Tris-HCl. Examples of the salt include 1 mM to 1 M sodium chloride, lithium chloride, and magnesium chloride. Examples of the reducing agent include 0.1 to 10 mM β-mercaptoethanol and dithiothreitol (DTT). Further, the protein solution may contain dimethylsulfoxide (DMSO) or ethylene glycol. The solution containing the protein has a pH of 4 to 11, preferably a pH of 6 to 9. Such protein solution or florigen solution may be used for crystallization without any further treatment, or as necessary, a preservative, a stabilizer, a surfactant, or the like is further added to the solution, and the resultant solution may be used for crystallization.

As a method of crystallizing a protein (polypeptide), a general technique for protein crystallization such as a vapor diffusion method, a batch method, or a dialysis method may be employed. Further, in the crystallization of a protein, it is important to determine physical and chemical factors such as the concentration of the protein, the concentration of a salt, a pH, the kind of a precipitant, and a temperature.

The vapor diffusion method refers to a method involving placing a droplet of a protein solution including a precipitant in a container including a buffer (external solution) containing the precipitant at a higher concentration, sealing the container, and then leaving the resultant to stand still. The vapor diffusion method is classified into a hanging drop method and a sitting drop method depending on how to place the droplet, and any of the methods may be adopted in the present invention. The hanging drop method is a method involving placing a small droplet of a protein solution on a cover glass, inverting the cover glass in a reservoir, and sealing the reservoir. On the other hand, the sitting drop method is a method involving installing an appropriate droplet stage in a reservoir, placing a droplet of a protein solution on the droplet stage, and sealing the reservoir with a cover glass or the like. In any of the methods, a precipitant is incorporated into the solution in the reservoir (reservoir solution). As appropriate, a small amount of the precipitant may be incorporated into a protein small droplet.

The reservoir solution (also referred to as precipitant solution) to be used in the vapor diffusion method is a solution formed of a buffer, a precipitant, a salt, and the like. Any buffer, precipitant, and salt may be used as long as a crystal can be efficiently produced. For example, the buffer is selected from 5 to 200 mM Na-HEPES, sodium phosphate, potassium phosphate, Tris-HCl, sodium acetate, citric acid, cacodylic acid, and the like at a pH of 4 to 10, the precipitant is selected from 5 to 35 vol% polyethylene glycol (PEG) having a molecular weight of 550 to 20,000, 0.2 to 2 M ammonium sulfate, 5 to 35 vol% methylpentanediol (MPD), 0.2 to 2 M ammonium tartrate, 5 to 35 vol% isopropanol, and a combination thereof, and the salt is selected from 0.2 to 4 M sodium chloride, lithium chloride, magnesium chloride, and the like. The components for the reservoir solution are not limited to those described above.

A crystal of a florigen activation complex may be produced as described below. A florigen and a 14-3-3 protein are mixed with each other and dialyzed against a 1 to 50 mM Tris-HCl buffer (pH 6.5 to 8.5) containing 10 to 50 mM NaCl to prepare a sample of a complex. The protein solution (protein concentration: 5 to 40 mg/mL) is mixed with a precipitant solution (0.05 to 0.1 M HEPES (pH 6.5 to 8.5), 0.01 to 0.4 M ammonium sulfate, and 15 to 30 vol% PEG (molecular weight: 2,000 to 4,000)), and the crystallization of a complex of the florigen and the 14-3-3 protein is performed by a sitting drop method under the condition of 4 to 20°C. After about 1 to 3 weeks, a single crystal of the complex of the florigen and the 14-3-3 protein is obtained. The obtained single crystal is collected and incubated with a precipitant solution (0.05 to 0.1 M HEPES (pH 6.5 to 8.5), 0.01 to 0.4 M (preferably 0.15 to 0.25 M) ammonium sulfate, 15 to 30 vol% (preferably 23 to 27 vol%) PEG (molecular weight: 2,000 to 4,000)) containing 15 to 30 vol% ethylene glycol and a 1 to 5 mM bZIP transcription factor for 10 to 20 minutes. Thus, a crystal of a florigen activation complex suitable for X-ray crystallographic analysis can be obtained.

The crystal of a florigen activation complex may also be produced as described below. A florigen and a 14-3-3 protein are mixed with each other at a molar ratio of 1:1 to 2 (more preferably 1:1.5) and dialyzed against a 10 mM Tris-HCl buffer (pH 7.5) containing 20 mM NaCl to prepare a sample of a complex. 1 µl of the protein solution (protein concentration: 10 mg/mL) is mixed with 1 µl of a precipitant solution (0.1 M HEPES (pH 7.5), 0.2 M ammonium sulfate, and 25 vol% PEG 3350), and the crystallization of a complex of the florigen and the 14-3-3 protein is performed by a sitting drop method under the condition of 4°C. After about 1 to 3 weeks, a single crystal of the complex of the florigen and the 14-3-3 protein is obtained. The obtained single crystal is collected and incubated with a precipitant solution ((0.1 M HEPES (pH 7.5), 0.2 M ammonium sulfate, 25 vol% PEG 3350) containing 25% ethylene glycol and a 2 mM bZIP transcription factor for 10 to 20 minutes (more preferably for 15 minutes). Thus, a crystal of a florigen activation complex can be obtained.

A crystal of a florigen may be produced as described below. A purified florigen is dialyzed against a 1 to 50 mM Tris buffer (pH 5.5 to 7.5) containing 10 to 50 mM NaCl and concentrated so as to achieve a concentration of 5 to 40 mg/mL. The resultant protein solution is mixed with a precipitant solution (0.01 to 0.5 M (preferably 0.05 to 0.15 M) cacodylic acid (pH 5.5 to 7.5), 0.01 to 0.5 M (preferably 0.15 to 0.25 M) ammonium tartrate, and 10 to 50 vol% (preferably 25 to 35 vol%) PEG (molecular weight: 5,000 to 12,000), and crystallization is performed by a sitting drop method under the condition of 4 to 20°C. After about 0.5 to 3 days (preferably about 1 day), a crystal of a florigen activation complex suitable for X-ray crystallographic analysis can be obtained.

The crystal of a florigen may be produced as described below. A purified florigen is dialyzed against a 10 mM Tris buffer (pH 7.5) containing 20 mM NaCl and concentrated so as to achieve a concentration of 5 mg/ml. 1 µl of the resultant protein solution is mixed with 1 µl of a precipitant solution (0.1 M cacodylic acid (pH 6.5), 0.2 M ammonium tartrate, and 30 vol% PEG 8000), and crystallization is performed by a sitting drop method under the condition of 4°C. After about 0.5 to 3 days (preferably about 1 day), a crystal of a florigen activation complex suitable for X-ray crystallographic analysis can be obtained.

### (X-ray crystallographic analysis)

X-ray crystallographic analysis is most commonly performed as a technique for clarifying a conformation of a protein (polypeptide). This technique involves crystallizing a protein, irradiating the crystal with a monochromatic X-ray, and clarifying conformational information on the protein based on the resultant X-ray diffraction image. The conformational information includes an electron density map and atomic coordinates, and the atomic coordinates may be acquired by analysis according to a method known in the art (D.E. McRee, Practical Protein Crystallography, Academic Press, San Diego (1993)).

The X-ray crystallographic analysis involves the steps of: irradiating a crystal with an X-ray to acquire diffraction data; analyzing the resultant diffraction data to acquire an electron density of a protein (polypeptide); and analyzing the resultant electron density to acquire atomic coordinates of the protein (polypeptide).

In the X-ray crystallographic analysis, through the use of an X-ray diffractometer in a laboratory or a large radiation facility (e.g., ESRF, APS, SPring-8, PF, ALS, CHESS, SRS, LLNL, SSRL, or Brookhaven), diffraction data is collected by oscillation photography or the like with a two-dimensional detector such as an imaging plate or a CCD camera, and an electron density may be obtained from the data to elucidate atomic coordinates.

A crystal of a protein often undergoes damage by irradiation with an X-ray, resulting in a deterioration in diffraction ability. Hence, it is preferred to perform high-resolution X-ray diffraction through low-temperature measurement. The low-temperature measurement refers to a method involving freezing a crystal by rapidly cooling to about -173°C, and collecting diffraction data in the state. In general, in the freezing of a crystal of a protein, a contrivance such as treatment in a solution containing a protectant (cryoprotectant) such as glycerol is made for the purpose of preventing the collapse of the crystal due to the freezing. A frozen crystal may be prepared, for example, by flash freezing involving directly immersing a crystal, which has been immersed in a preservative solution supplemented with a protectant, in liquid nitrogen.

A diffraction image collected by an X-ray diffraction experiment may be processed with data processing software to calculate diffraction intensities obtained by the indexing and integration of individual diffraction spots. Electron densities in a three-dimensional space are derived by performing inverse Fourier transform using the diffraction intensities and phase information of the diffraction spots. In a diffraction experiment, it is impossible in principle to measure phase information on each of the diffraction spots necessary for the calculation of the electron density. Hence, in order to obtain the electron density, the phase as lost information is estimated by a molecular replacement method, a heavy atom isomorphous replacement method, a multiwavelength anomalous dispersion method (MAD method), or a modified method thereof.

An electron density map is depicted based on the thus obtained electron density, and a three-dimensional model is constructed using software which operates in a graphics workstation in accordance with the electron density map. After the construction of the model, structural refinement is performed by a least-squares method or the like to give final atomic coordinates (conformational coordinates) of a protein.

### (Conformational information on florigen activation complex or florigen)

The atomic coordinates mean mathematical coordinates in which the positions of the atoms of the protein described above are expressed as three-dimensional coordinates. The atomic coordinates substantially mean a space configuration determined depending on distances between the respective molecules (atoms) which construct a chemical structure. When the space configuration is processed on a computer as information, a relative configuration is converted into numerical information as specific coordinates in a certain coordinate system (referred to as conversion to coordinates). This is processing necessary for convenience in performing computer processing, and it should be understood that the nature of the atomic coordinates is a configuration determined depending on distances between the respective molecules (atoms) as described above and is not coordinate values specified temporarily at the time of computer processing. Further, the atomic coordinates as used herein mean coordinates of individual atoms which construct a substance (such as a protein or an amino acid). Further, in the present disclosure, through the use of the conformational information represented by the electron density and atomic coordinates, atomic coordinates obtained by homology modeling or the like on a computer may also be obtained as derivatives for a protein having 40% or more homology to the polypeptide of the present disclosure.

### (Method of regulating flowering of plant)

The present invention encompasses a method of regulating flowering of a plant. The regulation of flowering means accelerating and/or delaying flowering. In more detail, the present invention relates to a method of regulating flowering of a plant, the method including suppressing the formation of a florigen activation complex by affecting any one or a plurality of binding sites, that is, by regulating binding in the binding sites between a florigen and a 14-3-3 protein, wherein the sites of the florigen are corresponding to M63, R64, P96, F103, and R132 in SEQ ID NO: 1.

The suppression of the formation of a florigen activation complex is exemplified by the introduction of a mutation into a binding site between a florigen and a 14-3-3 protein, and the feeding of a substance capable of suppressing binding between a florigen and a 14-3-3 protein to a plant body.

The gene encoding the florigen may be a gene encoding a protein (mutant protein) having a mutation in at least one binding site among sites corresponding to M63and the like in SEQ ID NO: 1. A gene encoding a mutant protein may be produced by introducing a mutation using a genetic engineering technique well-known to a person skilled in the art such as a site-directed mutagenesis method. For example, a gene encoding a mutant protein may be produced by combining a PCR reaction, a restriction enzyme reaction, a ligation reaction, and the like. Specifically, a kit such as a QuikChange™ Site-Directed Mutagenesis Kit (STRATAGENE) may be used. The (over)expression of each of those genes in a plant body, preferably in a desired plant tissue cells, more preferably in cell organelles allows binding between a florigen and a 14-3-3 protein and/or binding between a 14-3-3 protein and a bZIP transcription factor to be promoted, and allows flowering to be promoted (accelerated).

A method of (over)expressing a gene is not particularly limited. In general, however, there is adopted a known method such as introducing an isolated gene into plant cells in an expressible manner by a genetic engineering technique. The known method is exemplified by a method involving introducing a recombinant vector containing a desired gene into a host. Specific examples thereof may include a method utilizing infection with *Agrobacterium* bacteria, such as a protoplast co-culture method or a leaf disk method, a polyethylene glycol method, an electroporation method, a microinjection method, a particle gun method, a liposome method, and an introduction method using an appropriate vector system, and an optimum method has only to be selected from the methods depending on the kind of host cells. It should be noted that examples of the vector include, but not particularly limited to, a plasmid, a phage, and a cosmid, and the vector has only to be appropriately selected depending on the kind of host cells. Further, a wide range of kinds of plants may be used as the host cells. In addition, the host cells may be proliferative plant materials such as protoplasts, cells, calli, organ leaves, seeds, germs, pollens, egg cells, and zygotes, or may be parts of a plant body such as flowers, fruits, leaves, roots, or rooted cuttings.

The promotion of flowering means shortening a vegetative growth period and accelerating the formation of flowers. It is considered that this allows picking seasons of agricultural and horticultural crops to be shifted (generally accelerated), and allows an improvement in efficiency of breeding and increases in yield of agricultural and horticultural crops to be achieved. Further, abilities to form flowers and seeds are estimated to be normal, and hence plant seeds can be quickly produced.

The introduction of a gene expressing a mutant florigen into a plant body allows the formation of a florigen activation complex to be inhibited and allows flowering to be regulated. The "modification" as used herein means completely deleting any one of amino acid sequence regions characteristic of those proteins, and introducing deletions, substitutions, and/or insertions into one or several amino acid residues for a particular site of those sequence regions. The modified protein includes a mutant protein.

A method of modifying a gene encoding each protein has only to be performed by a conventionally known technique and is not particularly limited. That is, as mentioned above, the method has only to be performed, for example, by introducing a mutation into a base sequence utilizing a PCR method, by a known site-directed mutagenesis method (Kunkel et al.: Proc. Natl. Acad. Sci. USA, vol. 82. p488-(1985)), or with a commercially available kit (e.g., Quikchange Site-Directed Mutagenesis Kit: STRATAGENE).

The modified protein acts in a dominant manner on a protein obtained from a wild-type gene, and suppresses the transcription of a target gene to inhibit the expression of the target gene. Therefore, through the use of a modified gene, a function inherent in a wild-type gene is inhibited and lost, and a transformant in which flowering is suppressed can be produced. Hence, the time and effort required for the production of a transformant in which flowering is suppressed can be reduced.

A method of introducing a modified protein may be performed by the above-mentioned gene expression method.

Further, the introduction of a mutation into a binding site of an endogenous protein of a plant body, and the knockout of a protein may be performed by any of known methods utilizing an antisense method, a gene targeting method, a gene knockout tagging method, and the like (Kempin et al. Nature 389: 802-803. 1997).

The antisense method is a method involving constructing a vector containing an appropriate promoter and a target gene in a reverse direction (antisense DNA) disposed downstream of the promoter, and introducing the vector into a plant, thereby suppressing the expression of the target gene. The gene targeting method is a method involving inserting another DNA by homologous recombination into part of a target gene to knock out the target gene. The method of the present invention using the gene knockout tagging method is a method involving inserting T-DNA or a transposon into a genome at random and screening a target gene-knockout strain by utilizing PCR.

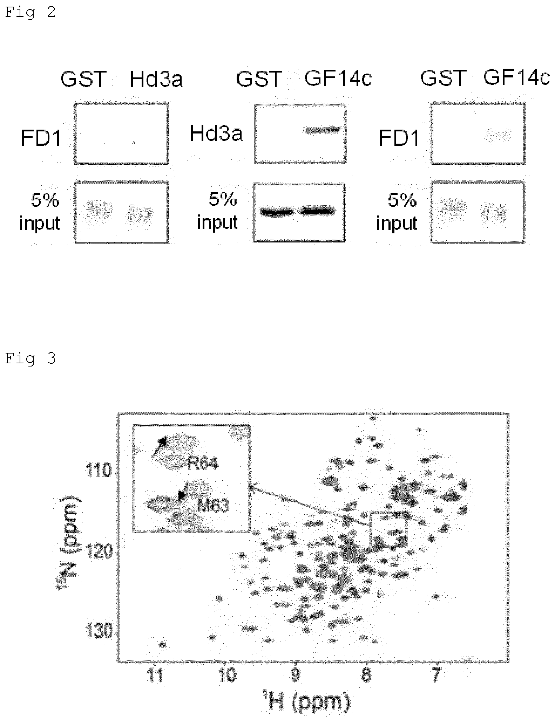

The suppression of flowering means extending a vegetative growth period and delaying the formation of flowers. It is considered that the suppression results in an increase in weight of a plant body as compared to a wild-type plant body (Development 135, 767-774 (2008) doi:10.1242/dev.008631, Fig 3E). That is, the suppression of flowering allows a plant body having an increased amount of biomass to be produced per individual to be provided. The increased amount of biomass to be produced means that a total weight per individual is large as compared to a wild-type, and also includes the case where the weight of part of tissues of a plant body is specifically large and the weights of the other tissues are similar to those of a wild-type. Plant biomass is generated by fixation of carbon dioxide in air using solar energy, and hence can be trapped as so-called carbon neutral energy. An increase in plant biomass has effects of global environment conservation, global warming prevention, and greenhouse gas emission reduction. Further, for a plant in which parts other than organs involved in flower-bud formation (flowers or seeds) are used as foods and the like, increases in yield of agricultural and horticultural crops per individual can be achieved by increasing the weight of a plant body through the suppression of flowering.

### (Transformant)

The present invention also encompasses a transformant having a desired gene introduced therein or a transformant having a desired gene knocked out. The transformant of the present invention includes one obtained in the method of regulating flowering of a plant. The "transformant" includes a cell, a tissue, an organ, and a protoplast as well as a plant individual. Further, organisms as targets of transformation are also not particularly limited and examples thereof may include various microorganisms (including *Escherichia coli* and the like) and plants. Further, animals and insects may also be used as targets of transformation by selecting promoters and vectors. Further, the transformant as used herein also includes a plant having introduced therein the gene according to the present invention or an offspring of the plant having the same properties as the plant, or a tissue thereof. Such transformant may be produced using the technique described in the above-mentioned "Method of regulating flowering of plant" section.

### Examples

Hereinafter, the present invention is specifically described by way of examples and experimental examples for further understanding of the present invention. However, it should be appreciated that the scope of the present invention is by no means limited by these examples and experimental examples. Further, all of Hd3a, GF14, and FD1 used in the following examples are proteins derived from rice. In the notation of the proteins, each of the proteins may be specified with or without the prefix "Os." However, all of the proteins used in the following examples are proteins derived from rice.

First, a production method for a plasmid used in examples is shown below. Full-length cDNAs for Hd3a (Os06g0157700), OsGF14 (OsGF14a: Os08g0480800, OsGF14b: Os04g0462500, OsGF14c: Os08g0430500, OsGF14d: Os11g0546900, OsGF14e: Os02g0580300, OsGF14f: Os03g0710800, OsGF14g: Os01g0209200, and OsGF14h: Os11g0609600), and OsFD1 (Os09g0540800) were cloned by RT-PCR. The coding regions were PCR-amplified by a conventional method and introduced into a pENTR/D-TOPO cloning vector (Invitrogen) to obtain entry clones.

The introduction of amino-acid substitutions or deletions into OsGF14, OsFD1, and Hd3a was performed by PCR with KOD FX DNA polymerase (TOYOBO). The PCR-amplified fragments were introduced into the pENTR/D-TOPO cloning vector to obtain entry clones.

pGII pUbq GW-T7 and pGII pUbq HA-GW were produced by inserting a ubiquitin promoter derived from maize, an NOS termination region (Miki and Shimamoto, Plant Cell Physiol. 45, 490 (2004).), and an attR recombination region (Nakagawa et al., 2007) with a T7 tag region or an HA tag region into a pGreen II vector (Hellens et al., Plant Mol. Biol. 42, 819 (2000).).

A pUbq:Hd3a-mCherry expression vector (Non Patent Literature 2) and pHd3a:Hd3a-GFP and prolC:Hd3a-GFP binary vectors (Tamaki et al., Science 316, 1033 (2007)) were produced based on the descriptions of the literatures. A prolC:Hd3a(R64G)-GFP binary vector and a prolC:Hd3a(R64G/R132A)-GFP binary vector were produced using mutant Hd3a cDNA as described in Tamaki et al., Science 316, 1033 (2007). In order to produce other vectors, a mutant Hd3a gene, a mutant OsGF14 gene, and a mutant OsFD1 gene were transformed into various vectors in pENTR D-TOPO vectors by a Gateway recombination method with Gateway BP clonase II (Invitrogen).

Those vectors may be included in pBTM116-GW and pVP16-GW in a yeast two-hybrid assay (Examples 8 and 9). Further, the vectors may be included in pGII pUbq GW-T7, pGII pUbq HA-GW, p35S-GFP-GW, p35S-mCerulean-GW, p35S-Vn-GW, p35S-Vc-GW, p35S- GW-Vn, p35S-GW-Vc, and pUbq-GW in a transient expression assay (Examples 11 and 12) and in a p2K-GW binary vector and a pANDA RNAi vector (Miki and Shimamoto 2004) in the production of a transgenic plant (Examples 10, 13-1, and 13-2. SV40 NLS was fused with GF14b and GF14e by PCR.

### (Example 1) Confirmation of interactions among three proteins

(1) Interactions among three proteins were confirmed by an in vitro GST pull-down assay.
   A plasmid having incorporated therein cDNA encoding full-length Hd3a (residues 1 to 179) set forth in SEQ ID NO: 1 and cDNA encoding full-length GF14c (residues 1 to 256) set forth in SEQ ID NO: 2 was subjected to restriction enzyme treatment, and fragments obtained by the restriction enzyme treatment were fused with a polynucleotide encoding GST and introduced into a vector. The vector was introduced into *Escherichia coli,* and a GST-fused GF14c protein or a GST-fused Hd3a protein was expressed in *Escherichia coli.* After the culture of *Escherichia coli, Escherichia coli* was harvested by centrifugation, and the GST-fused GF14c protein or the GST-fused Hd3a protein was isolated and purified from the lysis solution.
   10 nmol of each of the isolated and purified GST-fused GF14c protein and GST-fused Hd3a protein were incubated with 10 µl of a Glutathione Sepharose 4B resin (GE Healthcare) to bind each of the polypeptides to the resin. After that, the resin was washed with a phosphate buffer (pH 6.8) containing 50 mM KCl and 1 mM DTT to remove a free GST-fused protein unbound to the resin. The isolated and purified GST protein bound to the resin was used as a negative control. To three kinds of resins to which the GST-GF14c protein, the GST-Hd3a protein, and the GST protein were bound, 1 nmol of each of the isolated and purified proteins (OsFD1 having introduced therein a mutation of S192E in a region of positions 147 to 195 in SEQ ID NO: 3 and full-length Hd3a, both of which were not fused with GST) was added and the mixtures were incubated at room temperature for 15 minutes. After that, the resins were washed with a phosphate buffer (pH 6.8) containing 50 mM KCl and 1 mM DTT to remove a free GST-GF14c polypeptide unbound to the resins. Each of the bound proteins was eluted with a phosphate buffer (pH 6.8) containing 50 mM glutathione, 50 mM KCl, and 1 mM DTT and confirmed by SDS polyacrylamide electrophoresis.
   FIG. 2 shows the results. No interaction was observed between Hd3a and OsFD1 (FD1), whereas interactions were observed between Hd3a and GF14c and between GF14c and OsFD1, suggesting that Hd3a and OsFD1 were bound via GF14c.
(2) The interaction between Hd3a and GF14c was confirmed using NMR. A 15N stable isotope-labeled Hd3a protein solution was prepared with a 50 mM phosphate buffer (pH 6.8) containing 100 mM KCl, 1 mM DTT, and 7% deuterium oxide so as to achieve a final concentration of 0.2 mM. After that, 15N-HSQC NMR spectra of the following solutions were measured: the prepared Hd3a protein solution alone; and a mixed solution obtained by adding to the Hd3a protein solution a GF14c protein unlabeled with a stable isotope at a molar ratio of 1:0.5. The spectra were compared to each other. The NMR measurement was performed at a temperature of 30°C and performed using an AV500 NMR apparatus manufactured by Bruker.

FIG. 3 shows the results. The NMR spectrum of Hd3a changed through binding with GF14c. Thus, binding between Hd3a and GF14c was confirmed. Further, the binding was found to be site-specific because the shift of NMR signals was partially (e.g., Hd3a R64 and M63 in the expanded view of FIG. 3) observed.

### (Example 2) Production of crystals of florigen activation complex

A plasmid having incorporated therein cDNA encoding residues 6 to 170 in full-length Hd3a (residues 1 to 179) set forth in SEQ ID NO: 1 and cDNA encoding residues 1 to 235 in full-length GF14c (residues 1 to 256) set forth in SEQ ID NO: 2 was subjected to restriction enzyme treatment, and fragments obtained by the restriction enzyme treatment were fused with a polynucleotide encoding GST and introduced into a vector. The vector was introduced into *Escherichia coli.* A GST-fused Hd3a protein and GF14c protein were expressed in *Escherichia coli.* After the culture of *Escherichia coli*, *Escherichia coli* was harvested by centrifugation, and the proteins were purified from the lysis solution. After that, analysis was performed by an SDS-polyacrylamide electrophoresis method (SDS-PAGE). As a result, the proteins were each found to have a purity of 95% or more. A polypeptide encoding residues 187 to 195 in full-length OsFD1 (residues 1 to 195) set forth in SEQ ID NO: 3 was prepared by chemical synthesis.

The resultant Hd3a and GF14c were mixed with each other at a molar ratio of 1:1.5 and dialyzed against a 10 mM Tris-HCl buffer (pH 7.5) containing 20 mM NaCl to prepare a sample of a complex. The crystallization of the complex was performed at 4°C by a sitting drop method using a mixture of 1 µl of the protein solution (protein concentration: 10 mg/mL) and 1 µl of a precipitant solution (0.1 M HEPES (pH 7.5), 0.2 M ammonium sulfate, and 25% PEG 3350). After about 2 weeks, single crystals each measuring 0.1 by 0.1 mm were obtained. The obtained Hd3a-GF14c complex crystals were collected and incubated with the precipitant solution containing 25% ethylene glycol and a 2 mM OsFD1 polypeptide for 15 minutes to produce crystals of a florigen activation complex of Hd3a, GF14c, and OsFD1.

The resultant crystals were the following five kinds:
(florigen activation complex 1) a crystal having a space group of P1 and lattice constants of a=76.7 Å, b=96.6 Å, c=99.5 Å, α=68.2°, β=87.9°, and γ=77.9° at a resolution of 2.4 Å;
(florigen activation complex 2) a crystal having a space group of P1 and lattice constants of a=76.8 Å, b=97.3 Å, c=99.8 Å, α=68.1°, β=87.8°, and γ=77.9° at a resolution of 2.2 Å;
(florigen activation complex 3) a crystal having a space group of P1 and lattice constants of a=76.2 Å, b=96.1 Å, c=99.1 Å, α=68.2°, β=88.6°, and γ=77.8° at a resolution of 2.8 Å;
(florigen activation complex 4) a crystal having a space group of P6522 and lattice constants of a=129.0 Å, b=129.0 Å, c=342.0 Å, α=90°, β=90°, and γ=120° at a resolution of 2.85 Å; and
(florigen activation complex 5) a crystal having a space group of P4 and lattice constants of a=155.9 Å, b=155.9 Å, c=496.4 Å, α=90°, β=90°, and γ=90° at a resolution of 2.96 Å.

### (Example 3) X-ray structure analysis of crystals of florigen activation complex

The resultant crystals were measured for their X-ray diffraction images using a CCD detector in the BL5A beamline of a synchrotron radiation research facility Photon Factory. Atomic coordinates of a florigen activation complex were obtained based on the resultant diffraction images.

X-ray diffraction data was collected to perform the indexing of individual diffraction spots and the calculation of diffraction intensities. Phase angles were determined from the resultant diffraction intensities and search models by a molecular replacement method. Electron density maps were derived by inverse Fourier transform based on the diffraction intensities of the diffraction spots and the phase angles described above. Atomic coordinates were constructed based on the resultant electron density maps.

Specifically, the resultant data was processed using HKL2000 and scaled with SCALEPACK. As a search model, the crystal structure of a florigen was determined by a molecular replacement method. The resultant model was refined at 2.4 Å using CNS and REFMAC. After each refinement, the resultant model was corrected with an electron density map 2Fo-Fc map using COOT.

### (Example 6) Confirmation of binding abilities of various mutant proteins

An interaction was confirmed by the method described in Example 1 except that mutant Hd3a and mutant GF14c were used. Mutant Hd3a (R55A, M63A, R64A, P96L, F103A, R132A, and R168A) and mutant GF14c (F200A, I204A, E212A, Y215A, and R226A) were produced using a Quickchange site-directed mutagenesis kit (Stratagene) according to the protocol of the kit. It should be noted that "R55A" means a mutant Hd3a protein having a substitution of arginine (R) at position 55 to alanine (A) in Hd3a.

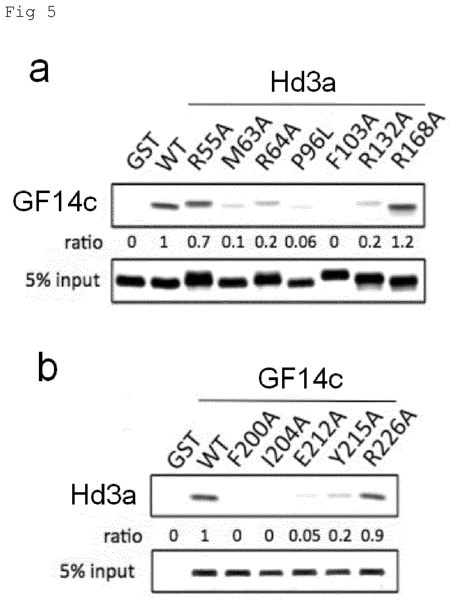

FIGS. 5 show the results. Out of the Hd3a mutants, M63A, R64A, P96L, F103A, and R132A each exhibited a reduction in binding ability to GF14c. In particular, F103A exhibited a remarkable reduction in binding ability (FIG. 5a). Out of the GF14c mutants, F200A, I204A, E212A, and Y215A each exhibited a reduction in binding ability to Hd3a. In particular, F200A and I204A each exhibited a remarkable reduction in binding ability (FIG. 5b).

### (Example 7) Gel-shift assay of florigen activation complex and C-box DNA

An *arabidopsis* API promoter-derived sequence (22 base pairs: 5'-CTTCACGAGACGTCGATAATCA-3' (SEQ ID NO: 5)) was used as C-box DNA. C-box DNA used for the assay was prepared by chemical synthesis. A 100 mM Tris-borate buffer containing 0.2 mM EDTA, 1.5 mM MgCl₂, and 5% glycerol was used for the preparation of a florigen activation complex. A conjugate of the florigen activation complex (FD1-GF14-Hd3a) (sometimes referred to as "FAC") and C-box DNA was generated by mixing 30 pmol of C-box DNA, 40 pmol of OsFD1, 40 pmol of GF14c, and 80 pmol of Hd3a with each other and subjecting the mixture to a reaction through incubation at 4°C for 30 minutes. The generated conjugate was subjected to electrophoresis using a 10% acrylamide gel and detected by ethidium bromide staining.

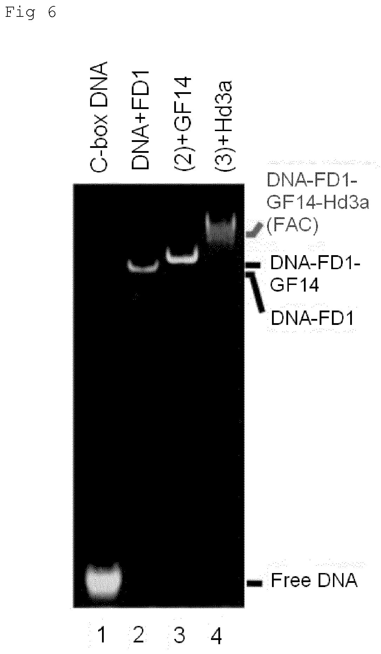

FIG. 6 shows the results. The florigen activation complex was found to bind to C-box DNA and form a stable complex on the DNA (lane 4). Hd3a is considered to form a florigen activation complex on a promoter and be responsible for the transcriptional activation of a gene necessary for flowering.

### (Example 8) Confirmation of interactions between florigen and 14-3-3 protein isoforms

Interactions between respective proteins were confirmed using a yeast two-hybrid assay. First, a plasmid was constructed as described below. Full-length cDNAs for OsGF14a (Os08g0480800), OsGF14b (Os04g0462500), OsGF14c (Os08g0430500), OsGF14d (Os11g0546900), OsGF14e (Os02g0580300), OsGF14f (Os03g0710800), OsGF14g (Os01g0209200), OsGF14h (Os11g0609600), and OsFD1 (Os09g0540800) were cloned by RT-PCR. The forward and reverse primers were designed based on genetic information in the rice DNA database. The full-length coding region was PCR-amplified and introduced into a pENTR/D-TOPO cloning vector (Invitrogen) to obtain entry clones.

The screening of Hd3a interactors was performed by the method described in Non Patent Literature 2. Yeast two-hybrid libraries were produced using total RNA extracted from wild-type leaf blades. After cDNA synthesis using a cDNA synthesis kit (Stratagene), the cDNAs were inserted into a pVP16 vector (Hollenberg et al., 1995) and introduced into a yeast L40 strain. As a bait, the full-length Hd3a ORF was cloned into a pBTM116 vector (Bartel et al., 1993). Screening was performed on an SC medium lacking histidine and containing 2.5 mM 3-aminotriazole (3-AT).

For an interaction assay, pBTM116 and pVP16 were converted to pBTM116-GW and pVP16-GW, respectively, using a Gateway vector conversion system (Invitrogen) according to the manufacturer's instructions. Yeast cells were grown at 30°C for 5 days using an SC medium (without uracil, tryptophan, leucine, and histidine), the medium containing added histidine or 1 to 10 mM 3-aminotriazole (+His or +3-AT). The concentration of 3-AT was determined by a bait-prey combination.

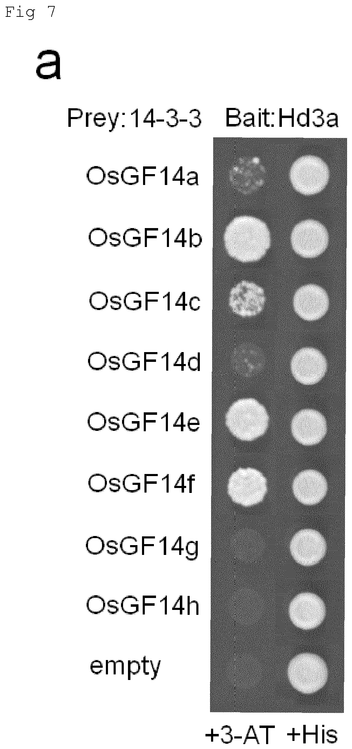

FIG. 7 shows the results. GF14b, c, e, and f (OsGF14b, c, e, and f) were each found to be exhibit an interaction with Hd3a. GF14b was used in the following examples.

### (Example 9) Confirmation of sites contributing to interactions among three proteins

Various mutant proteins were produced by constructing plasmids as described below. The introduction of amino-acid substitutions or deletions into OsGF14, OsFD1, and Hd3a was performed by PCR with KOD FX DNA polymerase (TOYOBO). The PCR-amplified fragments were cloned into a pENTR/D-TOPO cloning vector (Invitrogen) to obtain entry clones. Mutant proteins expressed in the obtained entry clones are as follows: S192A and S192E for OsFD1; R64A/R68A, I277A/L230A, F206A, I210A, E212A, E218A, Y221A, R232A, and D233A for OsGF14b; and R64G, R64A, R64K, T68I, P96L, F103A, R132A, R132K, R64G/R132A, and R64K/R132K for Hd3a.

Interactions were confirmed in the same manner as in Example 8 using those entry clones.

It should be noted that, out of the mutant proteins, a protein indicated using "/" is a multiple mutant protein, and for example, "R64G/R132A" for Hd3a means a double-mutant Hd3a protein having a substitution of arginine (R) at position 64 to glycine (G) and a substitution of arginine (R) at position 132 to alanine (A).

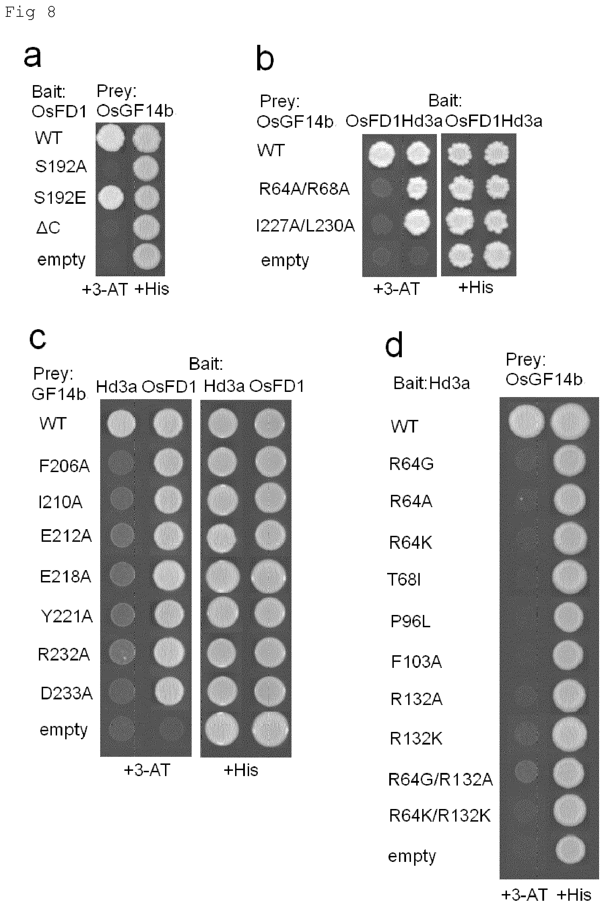

FIGS. 8a to 8d show the results.

The phosphorylation of serine at position 192 in OsFD1 was found to be important for an interaction between OsFD1 and OsGF14b (FIG. 8a).

Out of the mutant proteins for OsGF14b, in the mutant proteins having substitutions in F206, 1210, E212, E218, Y221, R232, and D233, there were reductions in binding ability to Hd3a, whereas there was no influence on binding ability to OsFD1 (FIG. 8c).

Out of the mutant proteins for Hd3a, in the mutant proteins having substitutions in R64, T68, P96, F103, and R132, there were remarkable reductions in binding ability to OsGF14b (FIG. 8d). Out of the mutant proteins for Hd3a, in the cases of P77L and R121H, there was no influence on binding ability to OsGF14b, suggesting that those sites were not important for binding to the 14-3-3 protein.

### (Example 10) Confirmation of interaction in shoot apex of rice

An interaction in the shoot apex of a rice plant was confirmed using an in vivo pull-down assay.

### (1) Used plant materials

Rice (*Oryza sativa* L. subsp. Japonica) variety Norin 8 was used as a wild-type. Transgenic rice (pHd3a::Hd3a::GFP, p35S::GFP, and prolC::Hd3a::GFP) were produced as described in Tamaki et al., Science 316, 1033 (2007) and Okano et al., Plant J. 53, 65 (2008). The transgenic rice was produced using *Agrobacterium*-mediated transformation of rice calli, as described in Hiei et al. Plant J. 6, 271 (1994), and a hygromycin-resistant plant was produced from the transformed calli. Transgene integration was further confirmed by PCR amplification of a hygromycin phosphotransferase (HPT) gene in genome DNA extracted from the produced plant. It should be noted that pHd3a::Hd3a::GFP refers to transgenic rice having introduced therein a gene in which a promoter region in Hd3a, a full-length coding region in Hd3a, and a coding region in GFP are conjugated to each other, and is hereinafter sometimes abbreviated as "HHG." p35S::GFP refers to transgenic rice having introduced therein a gene in which a CaMV 35S promoter and a coding region in GFP are conjugated to each other, and is hereinafter sometimes abbreviated as "35S GFP." prolC::Hd3a::GFP refers to transgenic rice having introduced therein a gene in which a promoter region in rolC, a full-length coding region in Hd3a, and a coding region in GFP are conjugated to each other.

### (3) In vivo pull-down assay

Samples of the shoot apical site were collected by microscopic dissection from HHG transgenic rice and 35S GFP transgenic rice. After grinding of the samples, the powder of each of the samples was dissolved in 100 µl of an extraction buffer (150 mM NaCl, 50 mM Tris, 0.1% Tween-20, 10% glycerol, 1 mM DTT, 1 mM Pefabloc SC (Roche), 1× Complete Proteinase Inhibitior Cocktail (Roche), and 1× Halt Phosphatase Inhibitor Cocktail (Pierce)), followed by mixing. After centrifugation (15 min, 4°C, 15,000 rcf), the supernatant was transferred to a tube, and the amount of a protein was measured by a Coomassie staining assay. An equivalent to the total amount of each of the proteins extracted from HHG transgenic rice and 35S GFP transgenic rice was incubated with 50 µl of anti-GFP MicroBeads (Miltenyi Biotec) according to the manufacturer's instructions except that the following modifications was made: a flow-through was collected and used for further analysis. The column was rinsed four times with 200 µl of an extraction buffer (150 mM NaCl, 50 mM Tris, 0.1% Tween-20, 10% glycerol, 1 mM DTT, 1 mM Pefabloc SC (Roche), 1× Complete Proteinase Inhibitior Cocktail (Roche), and 1× Halt Phosphatase Inhibitor Cocktail (Pierce)). After that, the column was washed once with 100 µl of a low ionic buffer (20 mM TrisHCl, pH 7.5). After that, the column was removed from the magnetic field, and the remaining protein was extracted with 50 µl of a lysis buffer. The eluate was separated by 12.5% SDS-PAGE and subjected to immunoblotting using a primary antibody (polyclonal rabbit anti-GFP antibody (Abcam)) and an anti-14-3-3 antibody (provided by Dr. Yohsuke Takahashi (Hiroshima University, Japan)). After washing with TBST, the membrane was incubated for 1 hour with anti-rabbit IgG conjugated to horseradish peroxidase (GE Healthcare). Detection was performed using enhanced chemiluminescence (ECL) protein gel blot detection reagents (GE Healthcare), and visualization was performed using an LAS-4000 mini Imager (Fujifilm).

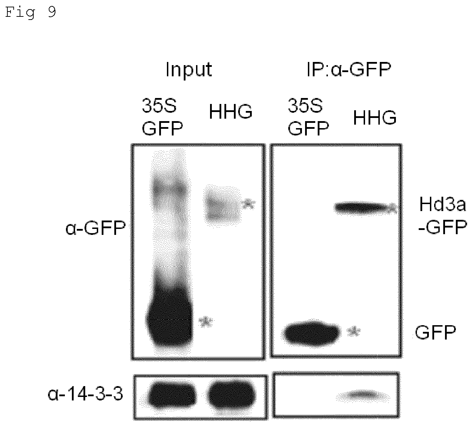

FIG. 9 shows the results. The left side (input) of FIG. 9 shows the results in the case of extracting a protein from shoot apex cells of transgenic rice expressing GFP or an Hd3a-GFP-fused protein (35S GFP transgenic rice or HHG transgenic rice) and subjecting the extract to immunoblotting using an anti-GFP antibody (a-GFP) and an anti-14-3-3 antibody (α-14-3-3). The right side (IP-GFP) of FIG. 9 shows the results in the case of subjecting an extract obtained by extracting a protein from shoot apex cells to immunoprecipitation (IP) with an anti-GFP antibody (a-GFP), followed by immunoblotting in the same manner as described above. When the Hd3a-GFP-fused protein was expressed (HHG extract), a 14-3-3 protein was observed during co-precipitation with the anti-GFP antibody. On the other hand, when GFP was expressed alone (35S GFP extract), a 14-3-3 protein was not observed during co-precipitation. This revealed that Hd3a interacted with the 14-3-3 protein in the shoot apex of rice.

### (Example 11) Subcellular localization of three proteins and complex thereof

Subcellular localization and bimolecular fluorescence complementation (BiFC method) were performed as described below.

First, Hd3a, GF14b, OsFD1, and β-glucuronidase (GUS) coding regions were cloned into fluorescent protein expression vectors or BiFC vectors and purified using a Purelink Plasmid Midiprep Kit (Invitrogen). For Hd3a, GF14b, OsFD1, and mutant proteins thereof, full-length coding regions were used. A vector expressing GFP, CFP, or mCherry was used as each of the fluorescent protein expression vectors. A vector expressing a Vn or Vc tag was used as each of the BiFC vectors. Vn or Vc emits fluoresce as Venus (mVenus) when exists in proximity.
(1) The transformation of rice Oc protoplasts was performed by a method described in Kyozuka and Shimamoto 1991, Plant J. 6, 271 (1994), Non Patent Literature 1, or the like. Fluorescent protein expression vectors having introduced therein various proteins were introduced into a protoplast suspension (2×10⁷ protoplasts/ml) by a PEG method (PEG-mediated method). After incubation at 30°C for 24 hours, transformed protoplasts were obtained and used for microscopic observation.
   In a co-expression system of two kinds of proteins, 1 µg of a GFP-GF14b expression plasmid or an Hd3a-mCherry expression plasmid and 10 µg of an NLS-CFP, CFP-OsFD1, or CFP-OsFD1 S192A expression plasmid were co-transformed into protoplasts. NLS refers to a nuclear localization signal peptide.

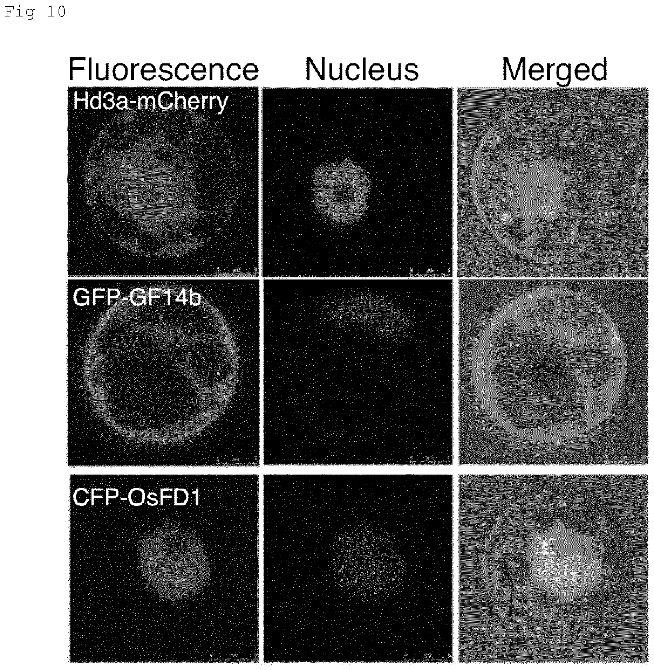

   FIG. 10 shows the results. Fluorescence from Hd3a-mCherry was detected in both the cytoplasm and the nucleus, fluorescence from GFP-GF14b was detected in the cytoplasm, and fluorescence from CFP-OsFD1 was detected in the nucleus.
(2) In the same manner as in the method of (1), 5 µg of a Vn-fused protein expression vector and a Vc-fused protein expression vector were co-transformed in the BiFC method.

An mCherry expression plasmid was introduced simultaneously as a marker for transformation efficiency.

In order to quantify protein-protein interactions, the fluorescence intensities of mCherry and Venus from about 20 cells of various protoplasts having introduced therein expression plasmids for BiFC analysis (a Vn-fused protein expression vector, a Vc-fused protein expression vector, and an mCherry expression plasmid) were measured under the same microscope settings, and a Venus/mCherry value was calculated. In the experimental settings, a BiFC signal in cells showing Venus/mCherry values of >0.33 for an interaction between Hd3a and GF14b and Venus/mCherry values >0.4 for an interaction between GF14 band OsFD1 and an interaction between Hd3a and OsFD1 was recognized as reliable one. Therefore, the number of cells showing higher values than the above-mentioned values was scored.

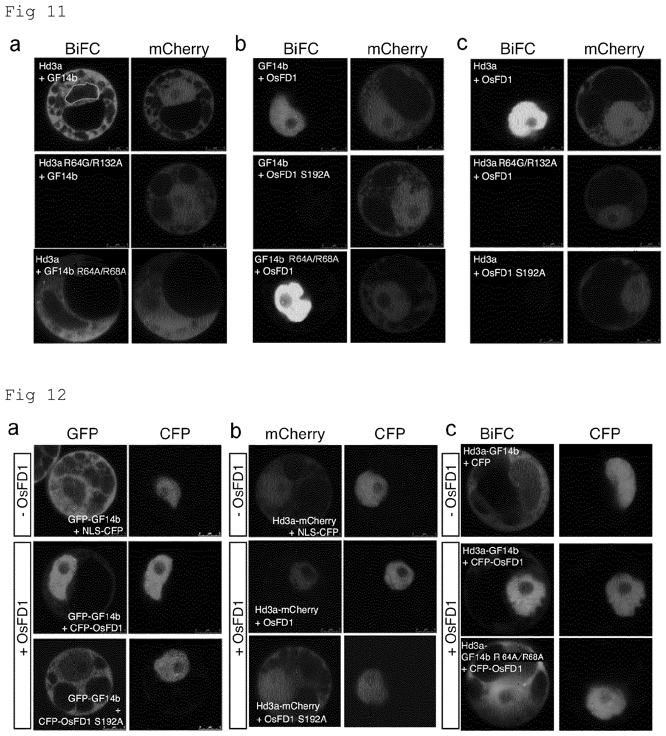

FIG. 11 shows the results.

Vn or Vc is conjugated to the N-terminal and the C-terminal of Hd3a and GF14b and expressed in rice protoplasts. As a result, BiFC signals were confirmed in the cytoplasm for the interaction between Hd3a and GF14b, which were dependent on mutations in R64 and R132 of Hd3a (FIG. 11a). In the BiFC method, mutations in R58 and R62 of GF14b did not affect the interaction between Hd3a and GF14b.

Next, an interaction between GF14b and OsFD1 was confirmed. As a result, the interaction therebetween was not observed in the cytoplasm. The interaction between GF14b and OsFD1 was detected mainly in the nucleus, and only the localization of GF14b was confirmed in the cytoplasm (FIG. 11b). A mutation in S192 of OsFD1 was found to inhibit the interaction with GF14b. Further, in the BiFC method, mutations in R58 and R132 of GF14b did not affect the interaction with OsFD1. An interaction between Hd3a and OsFD1 was confirmed. As a result, a complex of Hd3a and OsFD1 was found to be present in the nucleus (FIG. 11c). Mutations in R64 and R132 of Hd3a or a mutation in S192 of OsFD1 inhibited the interaction between Hd3a and OsFD1.
(3) In the same manner as in the method of (1), cells co-expressing OsFD1 in a BiFC system for confirming an interaction between Hd3a and GF14b were produced, and the amount of nuclear accumulation of the BiFC signal was examined. In order to co-express NLS-CFP and CFP-OsFD1 in the BiFC method, 10 µg of an NLS-CFP expression plasmid and a CFP-OsFD1 expression plasmid were used.

A method of calculating the amount of nuclear accumulation was established. First, the fluorescence intensities of Venus and mCherry in the nuclei of transformed cells were measured. After that, a value of (Veuns in nucleus/mCherry in nucleus)/(Venus in whole cell/mCherry in whole cell) was calculated. This value indicates a ratio between the amounts of nuclear accumulation of Venus and mCherry. Those values and the corresponding confocal images obtained from each cell were compared to each other. As compared to mCherry distribution, values of more than 1.2 were recognized as nuclear localization, values of 0.8 to 1.2 were recognized as localization in both the nucleus and the cytoplasm, and values of less than 0.8 were recognized as cytoplasmic localization.

FIG. 12 shows the results.

In the case of co-expression of GFP-GF14b with NLS-CFP, fluorescence from GFP was confirmed in the cytoplasm. In the case of transient co-expression of GFP-GF14b with CFP-OsFD1, fluorescence from CFP was confirmed in the nucleus, and fluorescence from GFP was also observed in the nucleus, which remarkably differed from the case of the co-expression with NLS-CFP. On the other hand, in the case of co-expression of an OsFD1 mutant protein S192A with GFP-GF14b, the movement of fluorescence from GFP into the nucleus was not observed (FIG. 12a). This suggested that the localization of GF14b was transferred from the nucleus to the cytoplasm owing to the presence of OsFD1.

In the case of co-expression of Hd3a-mCherry with CFP-OsFD1, the amount of nuclear accumulation of mCherry increased as compared to the case of the co-expression with CFP alone. In the case of co-expression of an OsFD1 mutant protein S192A with Hd3a-mCherry, no increase in the amount of nuclear accumulation was found (FIG. 12b). Those results suggest that Hd3a and GF14b move from the cytoplasm to the nucleus to form a complex with OsFD1.

In order to confirm the influence of OsFD1 on the localization of a complex of Hd3a and GF14b, the BiFC method and CFP-OsFD1 were co-expressed. When OsFD1 was not expressed, the complex of Hd3a and GF14b (BiFC signal) was found to be present in the cytoplasm. In addition, when CFP-OsFD1 is co-expressed, the complex of Hd3a and GF14b was clearly observed predominantly in the nucleus (FIG. 12c). The movement of the complex of Hd3a and GF14b by OsFD1 co-expression was reduced by introducing mutations R58A/R62A into GF14b. For the localization of each protein in rice cells, it was suggested that the complex of Hd3a and GF14b was first formed in the cytoplasm, and the complex of Hd3a and GF14b interacted with OsFD1 in the nucleus through an interaction between GF14b and OsFD1.

### (Example 12) Influence of interaction among OsFD1, GF14b, and Hd3a on OsMADS15 transcriptional control

The influence of interactions among OsFD1, GF14b, and Hd3a on OsMADS15 transcriptional control was confirmed using a transient expression assay using protoplasts.

The transformation of rice Oc protoplasts was performed by a method described in Kyozuka and Shimamoto 1991, Plant J. 6, 271 (1994), Non Patent Literature 1, or the like. 8 µg of Hd3a expression vectors and 16 µg of OsFD1 expression vectors were introduced into 500 µl of a protoplast suspension (2×10⁷ protoplasts/ml) by a PEG method (PEG-mediated method). After incubation at 30°C for 24 hours, the protoplast suspension was centrifuged and the cell pellet was frozen at -80°C for RNA extraction.

pGII pUbq GW-T7 and pGII pUbq HA-GW were produced by inserting a ubiquitin promoter derived from maize, an NOS termination region (Miki and Shimamoto, Plant Cell Physiol. 45, 490 (2004)), and an attR recombination region with a T7 tag region or an HA tag region (Nakagawa et al., 2007) into a pGreen II vector (Hellens et al., Plant Mol. Biol. 42, 819 (2000)).

An Hd3a gene, an OsFD1 gene, or a mutant gene thereof incorporated in a pENTR D-TOPO vector was transformed into pGII pUbq GW-T7 and pGII pUbq HA-GW by a Gateway recombination method with Gateway BP clonase II (Invitrogen) (pGIIpUbqHd3a-T7, pGIIpUbqHA-OsFD1, pGIIpUbqHA-OsFD1, and pGIIpUbqHd3a-T7). Various Hd3a expression vectors and OsFD1 expression vectors were introduced into protoplasts to express various proteins, and the RNA expression amount of OsMADS15 was confirmed. It should be noted that OsGF14 is generally constantly expressed in all cells, and hence it is considered to be impossible to confirm its effect even when introduced.

### (2) RNA extraction and real-time PCR analysis were performed as described below.

Leaf blades of wild-type and transgenic plants were harvested. Total RNA was extracted using a TRizol reagent (Invitrogen) according to the manufacturer's protocol. cDNA was synthesized from 1 or 2 µg of RNA using an oligo dT primer (21-mer) and reverse-transcribed with Superscript II reverse transcriptase (Invitrogen). 1 µl of cDNA was used for quantitative analysis of gene expression using SYBR Green PCR master mix (Applied Biosystems) with gene-specific primers. Data was collected using an ABI PRISM 7000 sequence detection system according to the manual. Primers for ubquitin and OsMADS15 are as described in Komiya et al., Development 135, 767 (2008). Primers for GF14b and GF14e are as follows:
GF14b-realF GCGGAAGAGATCAGGGAAG (SEQ ID NO: 4);
GF14b realR CGACAACAATCACAGCCACA (SEQ ID NO: 5);
GF14e realF CAAGGGCGAATCAGGAGA (SEQ ID NO: 6); and
GF14e realR TGAAACGATAACCCACAGCA (SEQ ID NO: 7).

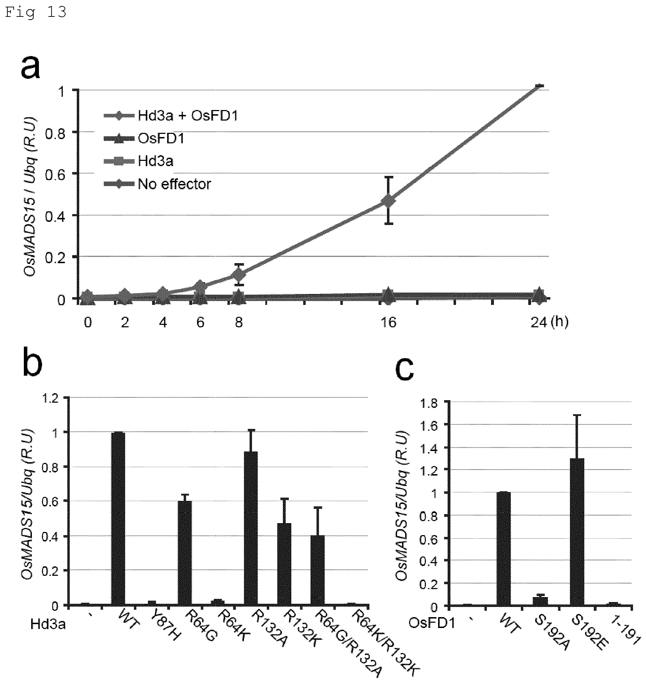

FIG. 13 shows the results.

The amount of OsMADS15 RNA increased with time after Hd3a and OsFD1 co-transformation. Hd3a or OsFD1 alone did not increase the RNA expression amount of OsMADS15. The OsMADS15 activation was found to start 4 hours after transformation, suggesting quick responsiveness between Hd3a and OsFD1 (FIG. 13a).

FIG. 13b shows the results of co-expression of various Hd3a mutants with OsFD1. Mutant Hd3a Y87H did not promote OsMADS15 transcription. Mutant Hd3a R64G and R132A slightly reduced OsMADS15 expression. Mutant Hd3a R64K and R64K/R132K remarkably reduced OsMADS15 expression. Those results suggest that the interaction between Hd3a and OsGF14 is required for OsMADS15 expression, and hydrophobicity, not a positive charge, of arginine is important for the interaction between Hd3a and OsGF14.

FIG. 13c shows the results of co-expression of various OsFD1 mutants with Hd3a. In the case where the SAP motif was deleted in OsFD1 ("1-191" in FIG. 13c: a fragment formed of residues 1-191, in which the subsequent amino acids were deleted) or in the case of a serine-to-alanine substitution in the SAP motif (S192A), the amount of OsMADS15 RNA accumulation was found to remarkably lower. In the case of a substitution of serine in the SAP motif to glutamic acid as a phospho-mimicking mutation (S192E), the amount of OsMADS15 RNA accumulation was not found to lower. Those facts suggest that the recognition of phosphorylated serine in the SAP motif in OsFD1 by the 14-3-3 protein is important for the formation of a florigen activation complex.

### (Example 13-1) Confirmation of flowering time in transgenic rice

In the same manner as in Example 10, transgenic rice was generated according to Table 3 and its flowering time was confirmed.

A plant was grown at a humidity of 70% in a chamber under short-day (SD) conditions (period including a light period at 30°C for 10 hours and a dark period at 25°C for 14 hours) or in a chamber under long-day (LD) conditions (period including a light period for 14 hours and a dark period for 10 hours). In the light period, photoirradiation was performed using a white-light fluorescent tube (400 to 700 nm, 100 µmol·m⁻²·s⁻¹). Rice suspension-cultured cells were maintained as described in Kyozuka and Shimamoto, 1991. A plant was grown at a humidity of 80% in a chamber under short-day (SD) conditions. The number of days to flowering was measured as the number of days to heading (heading stage) of a transgenic plant of T₀ generation after transferred to SD conditions.

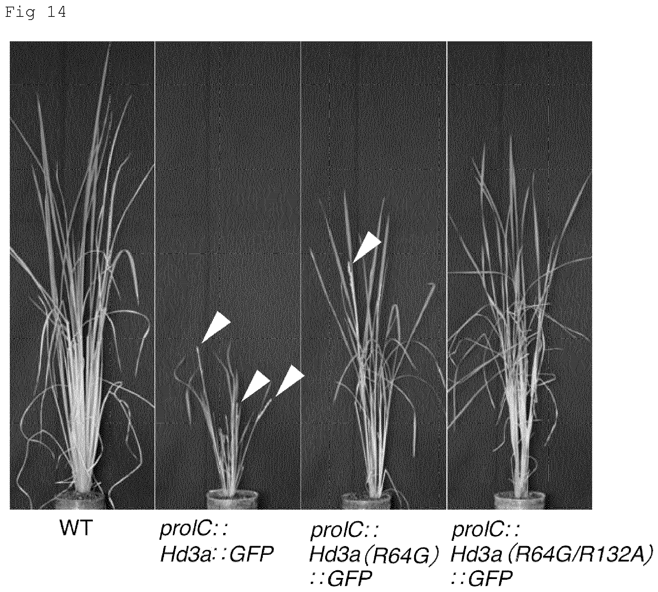

FIG. 14 and Table 3 show the results. An Hd3a-GFP protein derived from transgenic rice having introduced therein prolC::Hd3a::GFP moved from the shoot apex and strongly promoted flowering in the transgenic rice. prolC::Hd3a(R64G)::GFP (gene in which a promoter region in rolC, a coding region in R64G-mutant Hd3a, and a coding region in GFP were conjugated to each other) and prolC::Hd3a(R64G/R132A)::GFP (gene in which a promoter region in rolC, a coding region in R64G/R132A double-mutant Hd3a, and a coding region in GFP are conjugated to each other) were introduced into rice to confirm flowering time. As compared to transgenic rice having introduced therein prolC::Hd3a::GFP (19.6±12.3 days), transgenic rice having introduced therein prolC::Hd3a(R64G)::GFP (44.2±7.8 days) and transgenic rice having introduced therein prolC::Hd3a(R64G/R132A)::GFP (58.8±9.3 days) showed remarkably decreased promotion of flowering, and the latter was almost indistinguishable from a wild-type plant. Those facts suggest that an ability to promote flowering was lost by reduced affinity between Hd3a and 14-3-3.

Next, rice having introduced therein OsFD1 RNAi, transgenic rice expressing OsFD1, and transgenic rice having introduced therein mutant OsFD1 were analyzed. Rice having introduced therein OsFD1 RNAi flowered later than a wild-type. Transgenic rice expressing OsFD1 (pUbq::OsFD1) and transgenic rice having introduced therein mutant OsFD1 S192A (pUbq::OsFD1(S192A)) were not affected in terms of flowering, and transgenic rice having introduced therein mutant OsFD1 S192E (pUbq::OsFD1(S192E) (S192 phospho-mimicking)) promoted flowering as compared to a wild-type.

**[Table 3]**

| Genotype | Days to flowering (days) | n |
|---|---|---|
| Wild-type | 54.9 ± 9.7 | 8 |
| *prolC::Hd3a::GFP* | 19.6 ± 12.3 | 13 |
| *prolC::Hd3a(R64G)::GFP* | 44.2 ± 7.8 | 6 |
| *prolC::Hd3a(R64G*/*R132A)::GFP* | 58.8 ± 9.3 | 21 |
| Wild-type* (Wild-type) | 69.5 ± 6.0* | 4* |
| *OsFD1 RNAi** | 74.6 ± 4.8* | 7* |
| *pUbq::OsFD1* | 58.0 ± 6.1 | 3 |
| *pUbq::DsFD1*(*S192A*) | 61.7 ± 2.9 | 3 |
| *pUbq::OsFD1*(*S192E*) | 38.3 ± 4.6 | 8 |
| *GF14b GF14e double RNAi* | 60.1 ± 7.1 | 14 |
| *pUbq::GF14b* | 55.4 ± 10.9 | 5 |
| *pUbq::NLS::GF14b* | 51.0 ± 8.9 | 13 |
| *pUbq::GF14e* | 61.2 ± 11.0 | 6 |
| *pUbq::NLS::GF14e* | 47.7 ± 8.3 | 7 |

### (Example 13-2) Confirmation of flowering time in transgenic rice

Transgenic rice was generated in the same manner as in Example 13-1, and its flowering time was confirmed.

A plant (transgenic rice) was grown by the same technique as in Example 13-1, and the number of days to flowering was measured as the number of days to heading (heading stage) of a transgenic plant of T₀ generation after transferred to SD conditions.

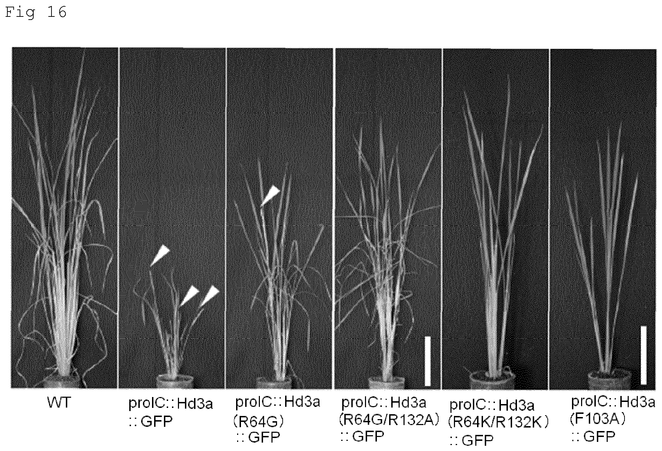

FIG. 16 and Table 4 show the results. In the same manner as in Example 13-1, in prolC::Hd3a::GFP transgenic rice, the Hd3a-GFP protein moved from the shoot apex and strongly promote flowering in the transgenic rice. As compared to transgenic rice having introduced therein prolC::Hd3a::GFP, the promotion of flowering was not found in transgenic rice having introduced therein prolC::Hd3a(R64G/R132K)::GFP and transgenic rice having introduced therein prolC:Hd3a(F103A)::GFP (gene in which a promoter region in rolC, a coding region in F103A-mutant Hd3a, and a coding region in GFP were conjugated to each other).

**[Table 4]**

| Genotype | Days to Flowering | n | P |
|---|---|---|---|
| WT | 55.7±9.4 | 9 | |
| prolC::Hd3a::GFP | 19.6±12.3** | 13 | 0.00000018 |
| prolC::Hd3aCR64G)::GFP | 44.2±7.8* | 6 | 0.024 |
| prolC::Hd3a(R64G/R132A)::GFP | 58.8±9.3 | 21 | 0.42 |
| prolC::Hd3a(R64K/R132K)::GFP | 61.9±9.7 | 15 | 0.13 |
| prolC::Hd3a(F103A)::GFP | 54.7±7.9 | 7 | 0.83 |
| *P<0.05, **P<0.01 | | | |

### Industrial Applicability

The present invention provides conformational information on the florigen activation complex important for the elucidation of a mechanism for controlling flowering of a plant, and may be used as a material for additional research on a mechanism for regulating flowering. Further, the flowering of a plant can be artificially and efficiently regulated based on the resultant conformational information, in particular, information on a binding site in the florigen activation complex. Based on the conformational information obtained in the present invention, a transgenic plant, which may be widely utilized for an increase in yield of an agricultural product, an improvement in efficiency of breeding, and the like, can be obtained.

## Claims

1. A method of regulating flowering of a plant, the method comprising suppressing formation of a florigen activation complex comprising a complex of a florigen, a 14-3-3 protein, and a bZIP transcription factor by generating a transformant comprising at least a florigen having a mutation in at least one binding site selected from the group consisting of sites of the florigen corresponding to M63, R64, P96, F103, and R132 in SEQ ID NO: 1.

2. A transformant, comprising at least a florigen having a mutation in at least one binding site between the florigen and a 14-3-3 protein, wherein:
the binding site between the florigen and the 14-3-3 protein comprises at least one site selected from the group consisting of sites of the florigen corresponding to M63, R64, P96, F103, and R132 in SEQ ID NO: 1.

3. A polynucleotide, which encodes at least a florigen having a mutation in at least one binding site between the florigen and a 14-3-3 protein; wherein:
the binding site between the florigen and the 14-3-3 protein comprises at least one site selected from the group consisting of sites of the florigen corresponding to M63, R64, P96, F103, and R132 in SEQ ID NO: 1.

4. A recombinant vector, comprising at least one of the polynucleotides of claim 3.

## Patentansprüche

1. Ein Verfahren zur Regulation der Blütezeit einer Pflanze, wobei das Verfahren die Unterdrückung der Bildung eines Florigen-Aktivierungskomplex umfasst, umfassend einen Komplex aus einem Florigen, einem 14-3-3 Protein, und einem bZIP Transkriptionsfaktor durch Erzeugung eines Transformanten, umfassend mindestens ein Florigen mit einer Mutation in mindestens einer Bindungsstelle, ausgewählt aus der Gruppe bestehend aus Stellen des Florigens die M63, R64, P96, F103, und R132 in SEQ ID Nr. 1 entsprechen.

2. Ein Transformant, umfassend mindestens ein Florigen mit Mutation in mindestens einer Bindungsstelle zwischen dem Florigen und einem 14-3-3 Protein, wobei: die Bindungsstelle zwischen dem Florigen und dem 14-3-3 Protein mindestens eine Stelle umfasst, ausgewählt aus der Gruppe bestehend aus Stellen für das Florigen die M63, R64, P96, F103, und R132 in SEQ ID Nr. 1 entsprechen.

3. Ein Polynukleotid, welches für ein Florigen mit Mutation in mindestens einer Bindungsstelle zwischen dem Florigen und einem 14-3-3 Protein codiert, wobei: die Bindungsstelle zwischen dem Florigen und dem 14-3-3 Protein mindestens eine Stelle umfasst, ausgewählt aus der Gruppe bestehend aus Stellen für das Florigen die M63, R64, P96, F103, und R132 in SEQ ID Nr. 1 entsprechen.

4. Ein rekombinanter Vektor, umfassend mindestens eines der Polynukleotide nach Anspruch 3.

## Revendications

1. Procédé de régulation de la floraison d'une plante, le procédé comprenant la suppression de la formation d'un complexe d'activation du florigène comprenant un complexe d'un florigène, d'une protéine 14-3-3, et d'un facteur de transcription bZIP par production d'un transformant comprenant au moins un florigène comportant une mutation dans au moins un site de liaison choisi dans le groupe constitué de sites du florigène correspondant à M63, R64, P96, F103, et R132 dans SEQ ID NO : 1.

2. Transformant, comprenant au moins un florigène comportant une mutation dans au moins un site de liaison entre le florigène et une protéine 14-3-3, dans lequel :
le site de liaison entre le florigène et la protéine 14-3-3 comprend au moins un site choisi dans le groupe constitué des sites du florigène correspondant à M63, R64, P96, F103, et R132 dans SEQ ID NO : 1.

3. Polynucléotide, qui code pour au moins un florigène comportant une mutation dans au moins un site de liaison entre le florigène et une protéine 14-3-3, dans lequel :
le site de liaison entre le florigène et la protéine 14-3-3 comprend au moins un site choisi dans le groupe constitué des sites du florigène correspondant à M63, R64, P96, F103, et R132 dans SEQ ID NO : 1.

4. Vecteur recombinant, comprenant au moins l'un des polynucléotides selon la revendication 3.


### SEQUENCE LISTING

<110> NATIONAL UNIVERSITY CORPORATION NARA INSTITUTE OF SCIENCE AND TECHNOLOGY
<120> Florigen activation complex and its cystal structure
<130> 10-1017 1786
<150> JP2010-061562
   <151> 2010-03-17
<160> 14
<170> PatentIn version 3.5
<210> 1
   <211> 179
   <212> PRT
   <213> Oryza sativa
<400> 1
   Met Ala Gly Ser Gly Arg Asp Arg Asp Pro Leu Val Val Gly Arg Val
   1               5                   10                  15
   Val Gly Asp Val Leu Asp Ala Phe Val Arg Ser Thr Asn Leu Lys Val
               20                  25                  30
   Thr Tyr Gly Ser Lys Thr Val Ser Asn Gly Cys Glu Leu Lys Pro Ser
           35                  40                  45
   Met Val Thr His Gln Pro Arg Val Glu Val Gly Gly Asn Asp Met Arg
       50                  55                  60
   Thr Phe Tyr Thr Leu Val Met Val Asp Pro Asp Ala Pro Ser Pro Ser
   65                  70                  75                  80
   Asp Pro Asn Leu Arg Glu Tyr Leu His Trp Leu Val Thr Asp Ile Pro
                   85                  90                  95
   Gly Thr Thr Ala Ala Ser Phe Gly Gln Glu Val Met Cys Tyr Glu Ser
               100                 105                 110
   Pro Arg Pro Thr Met Gly Ile His Arg Leu Val Phe Val Leu Phe Gln
           115                 120                 125
   Gln Leu Gly Arg Gln Thr Val Tyr Ala Pro Gly Trp Arg Gln Asn Phe
       130                 135                 140
   Asn Thr Lys Asp Phe Ala Glu Leu Tyr Asn Leu Gly Ser Pro Val Ala
   145                 150                 155                 160
   Ala Val Tyr Phe Asn Cys Gln Arg Glu Ala Gly Ser Gly Gly Arg Arg
                   165                 170                 175
   Val Tyr Pro
<210> 2
   <211> 256
   <212> PRT
   <213> Oryza sativa
<400> 2
   Met Ser Arg Glu Glu Asn Val Tyr Met Ala Lys Leu Ala Glu Gln Ala
   1               5                   10                  15
   Glu Arg Tyr Glu Glu Met Val Glu Tyr Met Glu Lys Val Ala Lys Thr
               20                  25                  30
   Val Asp Val Glu Glu Leu Thr Val Glu Glu Arg Asn Leu Leu Ser Val
           35                  40                  45
   Ala Tyr Lys Asn Val Ile Gly Ala Arg Arg Ala Ser Trp Arg Ile Val
       50                  55                  60
   Ser Ser Ile Glu Gln Lys Glu Glu Gly Arg Gly Asn Glu Glu His Val
   65                  70                  75                  80
   Thr Leu Ile Lys Glu Tyr Arg Gly Lys Ile Glu Ala Glu Leu Ser Lys
                   85                  90                  95
   Ile Cys Asp Gly Ile Leu Lys Leu Leu Asp Ser His Leu Val Pro Ser
               100                 105                 110
   Ser Thr Ala Ala Glu Ser Lys Val Phe Tyr Leu Lys Met Lys Gly Asp
           115                 120                 125
   Tyr His Arg Tyr Leu Ala Glu Phe Lys Thr Gly Ala Glu Arg Lys Glu
       130                 135                 140
   Ala Ala Glu Ser Thr Met Val Ala Tyr Lys Ala Ala Gln Asp Ile Ala
   145                 150                 155                 160
   Leu Ala Asp Leu Ala Pro Thr His Pro Ile Arg Leu Gly Leu Ala Leu
                   165                 170                 175
   Asn Phe Ser Val Phe Tyr Tyr Glu Ile Leu Asn Ser Pro Asp Lys Ala
               180                 185                 190
   Cys Asn Leu Ala Lys Gln Ala Phe Asp Glu Ala Ile Ser Glu Leu Asp
           195                 200                 205
   Thr Leu Gly Glu Glu Ser Tyr Lys Asp Ser Thr Leu Ile Met Gln Leu
       210                 215                 220
   Leu Arg Asp Asn Leu Thr Leu Trp Thr Ser Asp Leu Thr Glu Asp Gly
   225                 230                 235                 240
   Gly Asp Glu Val Lys Glu Ala Ser Lys Gly Asp Ala Cys Glu Gly Gln
                   245                 250                 255
<210> 3
   <211> 195
   <212> PRT
   <213> Oryza sativa
<400> 3
   Met Ala Met Glu Asp Asp Glu Asp Met Trp Ala Asn Thr Ser Ser Pro
   1               5                   10                  15
   Ser Ala Ser Pro Pro Arg Pro Arg Gly Phe Ile Ser Thr Ala Leu Ser
               20                  25                  30
   Leu Asn Ser Thr His Leu Gln Gly Leu Leu Pro Ser Ser Phe Val Asp
           35                  40                  45
   Ala Ala Ala Ser Pro Cys His Ala Ser Gly Asn Asn Asn Gly Gly Gly
       50                  55                  60
   Asp Gly Arg Asn Ala Ala Pro Met Ser Ser Ile Phe Phe Ala Ser Ala
   65                  70                  75                  80
   Ser Tyr His Gln Gln Gln His His Leu Pro Ala Pro Ala Pro Leu Asp
                   85                  90                  95
   Gly Ala Ile Leu Pro Ala Arg Arg Phe Gly Leu Asp Met Cys Ala Ala
               100                 105                 110
   Ala Ala Ala Ala Pro Ala Gly Val Pro Ala Ala Gly Asp Arg Arg Lys
           115                 120                 125
   Arg Arg Met Ile Lys Asn Arg Glu Ser Ala Ala Arg Ser Arg Ala Arg
       130                 135                 140
   Lys Gln Ala Arg Val Asn Asn Leu Glu Thr Glu Val Glu Gln Leu Lys
   145                 150                 155                 160
   Gln Glu Asn Lys Met Leu Arg Val Lys Tyr Glu Gln Leu Arg Lys Thr
                   165                 170                 175
   Val Glu Val Pro Val Pro Val Arg Arg Thr Leu Gln Arg Val Leu Ser
               180                 185                 190
   Ala Pro Phe
           195
<210> 4
   <211> 262
   <212> PRT
   <213> Oryza sativa
<400> 4
   Met Ser Ala Gln Ala Glu Leu Ser Arg Glu Glu Asn Val Tyr Met Ala
   1               5                   10                  15
   Lys Leu Ala Glu Gln Ala Glu Arg Tyr Glu Glu Met Val Glu Phe Met
               20                  25                  30
   Glu Lys Val Ala Lys Thr Val Asp Ser Glu Glu Leu Thr Val Glu Glu
           35                  40                  45
   Arg Asn Leu Leu Ser Val Ala Tyr Lys Asn Val Ile Gly Ala Arg Arg
       50                  55                  60
   Ala Ser Trp Arg Ile Ile Ser Ser Ile Glu Gln Lys Glu Glu Ser Arg
   65                  70                  75                  80
   Gly Asn Glu Asp Arg Val Thr Leu Ile Lys Asp Tyr Arg Gly Lys Ile
                   85                  90                  95
   Glu Thr Glu Leu Thr Lys Ile Cys Asp Gly Ile Leu Lys Leu Leu Glu
               100                 105                 110
   Ser His Leu Val Pro Ser Ser Thr Ala Pro Glu Ser Lys Val Phe Tyr
           115                 120                 125
   Leu Lys Met Lys Gly Asp Tyr Tyr Arg Tyr Leu Ala Glu Phe Lys Thr
       130                 135                 140
   Gly Ala Glu Arg Lys Asp Ala Ala Glu Asn Thr Met Val Ala Tyr Lys
   145                 150                 155                 160
   Ala Ala Gln Asp Ile Ala Leu Ala Glu Leu Pro Pro Thr His Pro Ile
                   165                 170                 175
   Arg Leu Gly Leu Ala Leu Asn Phe Ser Val Phe Tyr Tyr Glu Ile Leu
               180                 185                 190
   Asn Ser Pro Asp Arg Ala Cys Asn Leu Ala Lys Gln Ala Phe Asp Glu
           195                 200                 205
   Ala Ile Ser Glu Leu Asp Thr Leu Ser Glu Glu Ser Tyr Lys Asp Ser
       210                 215                 220
   Thr Leu Ile Met Gln Leu Leu Arg Asp Asn Leu Thr Leu Trp Thr Ser
   225                 230                 235                 240
   Asp Ile Ser Glu Asp Thr Ala Glu Glu Ile Arg Glu Ala Pro Lys Arg
                   245                 250                 255
   Asp Ser Ser Glu Gly Gln
               260
<210> 5
   <211> 22
   <212> DNA
   <213> Artificial Sequence
<220>
   <223> Synthesized oligo nucleotide based on Arabidopsis AP1 promoter
<400> 5
   cttcacgaga cgtcgataat ca 22
<210> 6
   <211> 19
   <212> DNA
   <213> Artificial Sequence
<220>
   <223> Primer used for PCR amplification
<400> 6
   gcggaagaga tcagggaag 19
<210> 7
   <211> 20
   <212> DNA
   <213> Artificial Sequence
<220>
   <223> Primer used for PCR amplification
<400> 7
   cgacaacaat cacagccaca 20
<210> 8
   <211> 18
   <212> DNA
   <213> Artificial Sequence
<220>
   <223> Primer used for PCR amplification
<400> 8
   caagggcgaa tcaggaga 18
<210> 9
   <211> 20
   <212> DNA
   <213> Artificial Sequence
<220>
   <223> Primer used for PCR amplification
<400> 9
   tgaaacgata acccacagca 20
<210> 10
   <211> 6
   <212> DNA
   <213> Artificial Sequence
<220>
   <223> C-box DNA
<400> 10
   gacgtc 6
<210> 11
   <211> 847
   <212> DNA
   <213> Oryza sativa
<400> 11
   tgcaccacac acagttcagc tagcagatca cctagctaga tagctgcctc tatcacagta    60
   tatttgctcc ctgcaacttg ctgctgctgc aatagctagc agctgcagct agtaagcaaa   120
   actataaacc ttcagggttt tttgcaagat cgatggccgg aagtggcagg gacagggacc   180
   ctcttgtggt tggtagggtt gtgggtgatg tgctggacgc gttcgtccgg agcaccaacc   240
   tcaaggtcac ctatggctcc aagaccgtgt ccaatggctg cgagctcaag ccgtccatgg   300
   tcacccacca gcctagggtc gaggtcggcg gcaatgacat gaggacattc tacacccttg   360
   tgatggtaga cccagatgca ccaagcccaa gtgaccctaa ccttagggag tatctacatt   420
   ggttggtcac tgatattcct ggtactactg cagcgtcatt tgggcaagag gtgatgtgct   480
   acgagagccc aaggccaacc atggggatcc accggctggt gttcgtgctg ttccagcagc   540
   tggggcgtca gacagtgtac gcgcccgggt ggcgtcagaa cttcaacacc aaggacttcg   600
   ccgagctcta caacctcggc tcgccggtcg ccgccgtcta cttcaactgc cagcgcgagg   660
   caggctccgg cggcaggagg gtctacccct agctaacgat gatcccgatc gatctgctgc   720
   atgctcacta tcatcatcca gcatgctata cattgcaggt tcagacaatt gaaatgattc   780
   tcgacacaca acatatatat gatggtgtaa ttaattatgc aattaaatag ctgagcaagg   840
   ctaaggt                                                             847
<210> 12
   <211> 1189
   <212> DNA
   <213> Oryza sativa
<400> 12
   gccgaacaaa aagcattcgc atccacgaga ccactcgaac ccgacccgcc tcgccgccgc      60
   cgccaccgaa gtaatccctt aattggtcaa aatgtctcgg gaggagaatg tctacatggc     120
   caagctggcc gagcaggctg aaaggtatga ggagatggtt gagtacatgg agaaggttgc     180
   aaagactgta gatgtggaag agctcactgt tgaggagcgc aacctcttgt ctgttgctta     240
   caagaatgtg attggtgccc gccgtgcctc ctggcgtatt gtctcatcca ttgaacagaa     300
   ggaggagggt cgtggcaatg aggaacatgt tactctgatc aaggagtacc gtggcaagat     360
   tgaagctgag ctgagcaaga tttgcgatgg tatcctgaag ttgcttgact cacaccttgt     420
   gccctcatct actgctgcag aatctaaggt gttttacctc aagatgaagg gtgattacca     480
   caggtacctt gcggaattta agactggtgc cgagagaaag gaagctgctg agagcacaat     540
   ggtggcttac aaggctgctc aggatattgc tctggcggat cttgctccca cccatcccat     600
   aaggcttgga ctggcactta acttctctgt gttctactac gagattctaa actctccaga     660
   caaggcttgc aaccttgcta agcaggcgtt tgacgaagcc atctccgagt tggataccct     720
   cggggaggag tcttacaagg acagcacttt gatcatgcag ctcctgaggg acaacttgac     780
   cctctggacc tctgacctca cggaggacgg tggtgatgag gtgaaagaag cctccaaggg     840
   cgacgcctgc gagggccagt aaaatgggaa gatcgatcga tcgatggctc cgcatgttat     900
   tggagaccat cgatttagat gcctcatgct gctgtcacca tgatggatgg attcttctcc     960
   tgttctacta gaatgttttt cttcctgtcc ccccttcctc tctcttctct ggtttttact    1020
   agggtggtag cggtcgaatt agttcttccc attgctttgc atttggtgct agtggtccgt    1080
   ctgggctgat tgttttcctc tggatatgac tctcgtgtgt gttgtctcca gatagtgttt    1140
   tattgagcaa tatttaaagt tgtcgtccac ctcctcgatg ttgtttgtc               1189
<210> 13
   <211> 588
   <212> DNA
   <213> Oryza sativa
<400> 13
   atggcgatgg aggacgacga ggacatgtgg gcgaacacga gcagccccag cgcgtcgccg 60
   ccgcggccga gggggttcat ctccaccgcg ctgagcctca actcgacgca cctccaaggc 120
   ctcctcccgt cgtccttcgt cgacgccgcc gcctcgccgt gccacgccag cggcaacaac 180
   aacggcggcg gcgacggccg caatgccgcg ccgatgtcgt ccatcttctt cgcctccgcg 240
   tcgtaccacc agcagcagca ccacctccct gcccccgcgc cgctcgacgg cgccatcctc 300
   cccgcgcgcc ggttcgggct cgacatgtgc gccgccgccg ccgccgcccc cgccggtgtc 360
   cccgccgccg gggaccgccg caagaggcgg atgatcaaga accgcgagtc cgcggcgagg 420
   tcccgcgcgc gcaagcaggc ccgcgtcaac aacctggaga ccgaggtgga gcagctcaag 480
   caggagaaca agatgctccg cgtcaagtac gagcagctga ggaagacggt ggaggtgccg 540
   gtgccggtga ggaggactct gcagagggtg ctctccgcgc cattctga 588
<210> 14
   <211> 1155
   <212> DNA
   <213> Oryza sativa
<400> 14
   cgcctccccc caaaatttcc gacgccagag cgcgaggaga cgcacacaga gacttggcat      60
   ttgtagagtt tttagattta tagatagcaa agatgtcggc acaggcggag ctttcccgtg     120
   aggagaatgt gtacatggcc aagctcgctg agcaagccga gaggtacgag gagatggtcg     180
   aattcatgga gaaggtggcc aagacggttg actctgagga gctcaccgtg gaggagcgca     240
   acctcctgtc tgttgcatac aagaatgtga ttggagcccg ccgtgcgtca tggcgcatta     300
   tctcctccat tgagcagaag gaggaaagcc gtggtaacga ggaccgtgtc acactcatca     360
   aggactaccg tggcaagatc gagactgagc tcaccaagat ttgcgacggc attctcaagc     420
   tgcttgaatc ccaccttgtc ccctcttcca ctgcccctga gtccaaggtc ttctacctca     480
   aaatgaaggg tgactactac aggtaccttg ccgaatttaa gaccggggct gagaggaagg     540
   atgctgctga gaataccatg gtggcataca aggctgctca ggacattgct ttggctgagc     600
   tgcctcctac tcatccaatt aggcttgggc tagctcttaa cttctcagtg ttctactatg     660
   agatcctcaa ctcgcctgat cgtgcttgca acctcgcaaa gcaggctttt gatgaggcca     720
   tctcggagct ggacaccctg agcgaggagt cctacaagga cagcactttg atcatgcaac     780
   tcctccgtga taacctgacc ctgtggactt cagacatctc ggaggacacc gcggaagaga     840
   tcagggaagc tccgaagcgc gactccagcg aggggcagta aagccggctt tatgtgccct     900
   agaagcttgt agctagtgct ttgctactgt gtaatgacac ctatgtggct gtgattgttg     960
   tcgggaaatc tggggctccc ccgtatgtga ggttgctagc gatggttttg cagtctcgcc    1020
   tttaagctac tcgtagcaga gcaggtgggg gtctgtggag ccaggcctgg ttgggggtgg    1080
   gggagcctct tgaactgctt ggtggcactt cctgtttttc tgtttgcctt tttattggtg    1140
   tttattgtta tggtc                                                     1155